# NEW EUROPEAN PATENT SPECIFICATION

(11) **EP 0 255 378 B2**
(45) Date of publication and mention of the opposition decision: **06.02.2002**
(45) Mention of the grant of the patent: 13.04.1994
(21) Application number: 87306739.1
(22) Date of filing: 30.07.1987
(51) Int. Cl.: C12N 15/82, A01H 1/00, A01H 5/00

(54) **Seed specific transcriptional regulation**
Samenspezifische Transkriptionsregulierung
Régulation de transcription spécifique pour des graines

(30) Priority: 31.07.1986 US 891529; 28.07.1987 US 78538
(43) Date of publication of application: 03.02.1988
(73) Proprietor: Calgene LLC, Davis, California 95616 (US)
(72) Inventor: Kridl, Jean C., Davis California 95616 (US); Knauf, Vic C., Davis California 95616 (US)
(74) Representative: Paget, Hugh Charles Edward

(56) References cited:
- EP-A- 0 142 924
- WO-A-85/04899
- WO-A-87/07299
- PROC. NATL. ACAD. SCI. USA, vol. 82, May 1985, pages 3320-3324; C. SENGUPTA-GOPALAN et al.: "Developmentally regulated expression of the bean beta-phaseolin gene in tobacco seed"
- JOURNAL OF THE AMERICAN OIL CHEMISTS' SOCIETY, vol. 64, no. 5, May 1987,abstracts from Meeting Issue, AOCS, New Orleans, 17th-21st May 1987, page 633,abstract no. 55; V.C. KNAUF: "Genetic engineering of ACP levels in rapeseed"
- CHEMICAL ABSTRACTS, vol. 105, 1986, page 164, abstract no. 55584k, Columbus,Ohio, US; J.B. OHLROGGE et al.: "Spinach acyl-carrier proteins: structure,regulation, and progress towards cloning"
- COMMONWEALTH AGRICULTURAL BUREAU, abstract no. CAB 881669878; S. RADKE et al.:"Development of a transformation system for Brassica napus using Agrobacteriumbinary vectors"
- J. CELL. BIOCHEM. SUPPL., Part 9C, 1985, page 222, abstract no. 1695; S.R.SCOFIELD et al.: "Analysis of Brassica Napus storage protein genes"
- Wilmitzer (1988), Trends in Genetics, 4:13-18.
- Chen et al. (1988), P.N.A.S. USA, 83:8560-8564.
- Goldberg et al. (1989), 56:149-160.

## Description

### INTRODUCTION

### Technical Field

Genetic modification of plant material is provided for seed specific transcription. Production of endogenous products may be modulated or new capabilities provided.

### Background

The primary emphasis in genetic modification has been directed to prokaryotes and mammalian cells. For a variety of reasons plants have proven more intransigent than other eukaryotic cells in the ability to genetically manipulate the plants. In part, this has been the result of the different goals involved, since for the most part plant modification has been directed to modifying the entire plant or a particular plant part in a live plant, as distinct from modifying cells in culture.

For many applications, it will be desirable to provide for transcription in a particular plant part or at a particular time in the growth cycle of the plant. Toward this end, there is a substantial interest in identifying endogenous plant products whose transcription or expression are regulated in a manner of interest. In identifying such products, one must first look for products which appear at a particular time in the cell growth cycle or in a particular plant part, demonstrate its absence at other times or in other parts, identify nucleic acid sequences associated with the product and then identify the sequence in the genome of the plant in order to obtain the 5'-untranslated sequence associated with transcription. This requires substantial investigation in first identifying the particular sequence, followed by establishing that it is the correct sequence and isolating the desired transcriptional regulatory region. One must then prepare appropriate constructs, followed by demonstration that the constructs are efficacious in the desired manner.

Identifying such sequences is a challenging project, subject to substantial pitfalls and uncertainty. There is, however, substantial interest in being able to genetically modify plants, which justifies the substantial expenditures and efforts in identifying transcriptional sequences and manipulating them to determine their utility.

### Relevant Literature

Crouch et al., In: Molecular Form and Function of the Plant Genome, eds van Vloten-Doting, Groot and Hall, Plenum Publishing Corp. 1985, pp 555-566; Crouch and Sussex, Planta (1981) 153:64-74; Crouch et al., J. Mol. Appl. Genet. (1983) 2:273-283; and Simon et al., Plant Molecular Biology (1985) 5: 191-201, describe various aspects of Brassica napus storage proteins. Beachy et al., EMBO J. (1985) 4:3047-3053; Sengupta-Gopalan et al., Proc. Natl. Acad. Sci. USA (1985) 82:3320-3324; Greenwood and Chrispeels, Plant Physiol. (1985) 79:65-71 and Chen et al., Proc. Natl. Acad. Sci. USA (1986) 83:8560-8564 describe studies concerned with seed storage proteins and genetic manipulation. Eckes et al., Mol. Gen. Genet. (1986) 205:14-22 and Fluhr et al., Science (1986) 232:1106-1112 describe the genetic manipulation of light inducible plant genes.

Sengupta-Gopalan et al (1985), PNAS USA, 82, pp. 3320-3324, describes expression of an entire heterologous seed specific gene, phaseolin, in tobacco, under the regulatory control of the transcription initiation region which naturally provides for expression of that gene.

EP-A-0142924 (Agrigenetics Research Limited) describes a plasmid containing phaseolin 5' and 3' regulatory regions with a heterologous gene inserted between these regions. The aim and teaching presented by this document is to confer insects resistance by expressing the Bt protein in plants.

Scofield et al (1985), J. Cell Biochem. Suppl. 9c, abstract number 1695, discloses the isolation of genomic clones coding for napin.

### SUMMARY OF THE INVENTION

DNA constructs are provided which are employed in manipulating plant cells to provide for seed-specific transcription. Particularly, storage protein transcriptional regions are joined to other than the wild-type gene and introduced into plant genomes to provide for seed-specific transcription. The constructs provide for modulation of endogenous products as well as production of heterologous products.

In one aspect, the present invention provides DNA constructs employing a napin transcriptional initiation region to regulate expression of a DNA sequence of interest.

In another aspect, the present invention provides methods of modifying the genotype of a plant to impart a desired characteristic to seed as distinct from other plant tissues by means of such a DNA construct, and growing the plant to produce seed.

In a further aspect, the present invention provides DNA constructs employing a seed specific transcriptional initiation region to regulate expression of an acyl carrier protein.

In yet another aspect, the present invention provides methods for modifying the genotype of a plant to impart a desired characteristic to seed as distinct from other plant tissues using a seed specific transcriptional initiation region to regulate expression of an acyl carrier protein, and growing the plant to produce seed.

These aspects of the invention as more particularly set forth in the appended claims, even though the following description may in places have a broader scope than the claims.

### DESCRIPTION OF THE SPECIFIC EMBODIMENTS

Novel DNA constructs are provided which allow for modification of transcription in seed, particularly in embryos during seed maturation. The DNA constructs comprise a regulated transcriptional initiation region associated with seed formation, preferably in association with embryogenesis and seed maturation. Of particular interest are those transcriptional initiation regions associated with storage proteins, such as napin, cruciferin, β-conglycinin, or the like. The transcriptional initiation regions may be obtained from any convenient host, particularly plant hosts such as Brassica, e.g. napus or campestris, soybean (Glycine max), bean (Phaseolus vulgaris), corn (Zea mays), cotton (Gossypium sp.), safflower (Carthamus tinctorius), tomato (Lycopersican esculentum), and Cuphea species.

Downstream from and under the transcriptional initiation regulation of the seed specific region will be a sequence of interest which will provide for modification of the phenotype of the seed, by modulating the production of an endogenous product, as to amount, relative distribution, or the like, or production of a heterologous expression product to provide for a novel function or product in the seed. The DNA construct will also provide for a termination region, so as to provide an expression cassette into which a gene may be introduced. Conveniently, transcriptional initiation and termination regions may be provided separated in the direction of transcription by a linker or polylinker having one or a plurality of restriction sites for insertion of the gene to be under the transcriptional regulation of the regulatory regions. Usually, the linker will have from 1 to 10, more usually from about 1 to 8, preferably from about 2 to 6 restriction sites. Generally, the linker will be fewer than 100 bp, frequently fewer than 60 bp and generally at least about 5 bp.

The transcriptional initiation region may be native or homologous to the host or foreign or heterologous to the host. By foreign is intended that the transcriptional initiation region is not found in the wild-type host into which the transcriptional initiation region is introduced.

Transcriptional initiation regions of particular interest are those associated with the Brassica napus or campestris napin genes, acyl carrier proteins, genes that express from about day 7 to day 40 in seed, particularly having maximum expression from about day 10 to about day 20, where the expressed gene is not found in leaves, while the expressed product is found in seed in high abundance.

The transcriptional cassette will include in the 5'-3' direction of transcription, a transcriptional and translational initiation region, a sequence of interest, and a transcriptional and translational termination region functional in plants. One or more introns may also be present. The DNA sequence may have any open reading frame encoding a peptide of interest, e.g. an enzyme, or a sequence complementary to a genomic sequence, where the genomic sequence may be an open reading frame, an intron, a non-coding leader sequence, or any other sequence where the complementary sequence will inhibit transcription, messenger RNA processing, e.g. splicing, or translation. The DNA sequence of interest may be synthetic, naturally derived, or combinations thereof. Depending upon the nature of the DNA sequence of interest, it may be desirable to synthesize the sequence with plant preferred codons. The plant preferred codons may be determined from the codons of highest frequency in the proteins expressed in the largest amount in the particular plant species of interest.

In preparing the transcription cassette, the various DNA fragments may be manipulated, so as to provide for the DNA sequences in the proper orientation and, as appropriate, in the proper reading frame. Toward this end, adapters or linkers may be employed for joining the DNA fragments or other manipulations may be involved to provide for convenient restriction sites, removal of superfluous DNA, removal of restriction sites, or the like. For this purpose, in vitro mutagenesis, primer repair, restriction, annealing, resection, ligation, or the like may be employed, where insertions, deletions or substitutions, e.g. transitions and transversions, may be involved.

The termination region which is employed will be primarily one of convenience, since the termination regions appear to be relatively interchangeable. The termination region may be native with the transcriptional initiation region, may be native with the DNA sequence of interest, or may be derived from another source. Convenient termination regions are available from the Ti-plasmid of A. tumefaciens, such as the octopine synthase and nopaline synthase termination regions.

By appropriate manipulations, such as restriction, chewing back or filling in overhangs to provide blunt ends, ligation of linkers, or the like, complementary ends of the fragments can be provided for joining and ligation.

In carrying out the various steps, cloning is employed, so as to amplify the amount of DNA and to allow for analyzing the DNA to ensure that the operations have occurred in a proper manner. A wide variety of cloning vectors are available, where the cloning vector includes a replication system functional in E. coli and a marker which allows for selection of the transformed cells. Illustrative vectors include pBR332, pUC series, M13mp series, pACYC184, etc. Thus, the sequence may be inserted into the vector at an appropriate restriction site(s), the resulting plasmid used to transform the E. coli host, the E. coli grown in an appropriate nutrient medium and the cells harvested and lysed and the plasmid recovered. Analysis may involve sequence analysis, restriction analysis, electrophoresis, or the like. After each manipulation the DNA sequence to be used in the final construct may be restricted and joined to the next sequence, where each of the partial constructs may be cloned in the same or different plasmids.

In addition to the transcription construct, depending upon the manner of introduction of the transcription construct into the plant, other DNA sequences may be required. For example, when using the Ti- or Ri-plasmid for transformation of plant cells, as described below, at least the right border and frequently both the right a left borders of the T-DNA of the Ti-and Ri-plasmids will be joined as flanking regions to the transcription construct. The use of T-DNA for transformation of plant cells has received extensive study and is amply described in EPA Serial No. 120,516, Hoekema, In: "The Binary Plant Vector System", Offset-drukkerij Kanters B.V., Amsterdm, 1985, Chapter V, Fraley, et al., Crit. Rev. Plant Sci., 4:1-46, and An et al., EMBO J. (1985) 4:277-284.

Alternatively, to enhance integration into the plant genome, terminal repeats of transposons may be used as borders in conjunction with a transposase. In this situation, expression of the transposase should be inducible, or the transposase inactivated, so that once the transcription construct is integrated into the genome, it should be relatively stably integrated and avoid hopping.

The transcription construct will normally be joined to a marker for selection in plant cells. Conveniently, the marker may be resistance to a biocide, particularly an antibiotic, such as kanamycin, G418, bleomycin, hygromycin, chloramphenicol, or the like. The particular marker employed will be one which will allow for selection of transformed cells as compared to cells lacking the DNA which has been introduced.

A variety of techniques are available for the introduction of CNA into a plant cell host. These techniques include transformation with Ti-DNA employing A. tumefaciens or A. rhizogenes as the transforming agent, protoplast fusion, injection, electroporation, etc. For transformation with Agrobacterium, plasmids can be prepared in E. coli which plasmids contain DNA homologous with the Ti-plasmid, particularly T-DNA. The plasmid may or may not be capable of replication in Agrobacterium, that is, it may or may not have a broad spectrum prokaryotic replication system, e.g. RK290, depending in part upon whether the transcription construct is to be integrated into the Ti-plasmid or be retained on an independent plasmid. By means of a helper plasmid, the transcription construct may be transferred to the A. tumefaciens and the resulting transformed organism used for transforming plant cells.

Conveniently, explants may be cultivated with the A. tumefaciens or A. rhizogenes to allow for transfer of the transcription construct to the plant cells, the plant cells dispersed in an appropriate selective medium for selection, grown to callus, shoots grown and plantlets regenerated from the shoots by growing in rooting medium. The Agrobacterium host will contain a plasmid having the vir genes necessary for transfer of the T-DNA to the plant cells and may or may not have T-DNA. For injection and electroporation, disarmed Ti-plasmids (lacking the tumor genes, particularly the T-DNA region) may be introduced into the plant cell.

The constructs may be used in a variety of ways. Particularly, the constructs may be used to modify the fatty acid composition in seeds, that is changing the ratio and/or amounts of the various fatty acids, as to length, unsaturation, or the like. Thus, the fatty acid composition may be varied, enhancing the fatty acids of from 10 to 14 carbon atoms as compared to the fatty acids of from 16 to 18 carbon atoms, increasing or decreasing fatty acids of from 20 to 24 carbon atoms, providing for an enhanced proportion of fatty acids which are saturated or unsaturated, or the like. These results can be achieved by providing for reduction of expression of one or more endogenous products, particularly enzymes or cofactors, by producing a transcription product which is complementary to the transcription product of a native gene, so as to inhibit the maturation and/or expression of the transcription product, or providing for expression of a gene, either endogenous or exogenous, associated with fatty acid synthesis. Expression products associated with fatty acid synthesis include acyl carrier protein, thioesterase, acetyl transacylase, acetyl-coA carboxylasem, ketoacyl-synthases, malonyl transacylase, stearoyl-ACP desaturase, and other desaturase enzymes.

Alternatively, one may wish to provide various products from other sources including mammals, such as blood factors, lymphokines, colony stimulating factors, interferons, plasminogen activators, enzymes, e.g. superoxide dismutase, chymosin, etc., hormones, rat mammary thioesterase 2, phospholipid acyl desaturases involved in the synthesis of cicosapentaenoic acid, human serum albumin. Another purpose is to increase the level of seed proteins, particularly mutated seed proteins, having an improved amino acid distribution which would be better suited to the nutrient value of the seed. In this situation, one might provide for inhibition of the native seed protein by producing a complementary DNA sequence to the native coding region or non-coding region, where the complementary sequence would not efficiently hybridize to the mutated sequence, or inactivate the native transcriptional capability.

The cells which have been transformed may be grown into plants in accordance with conventional ways. See, for example, McCormick et al., Plant Cell Reports (1986) 5:81-84. These plants may then be grown, and either pollinated with the same transformed strain or different strains, identifying the resulting hybrid having the desired phenotypic characteristic. Two or more generations may be grown to ensure that the subject phenotypic characteristic is stably maintained and inherited and then seeds harvested to ensure the desired phenotype or other property has been achieved.

As a host cell, any plant variety may be employed which provides a seed of interest. Thus, for the most part, plants will be chosen where the seed is produced in high amounts or a seed specific product of interest is involved. Seeds of interest include the oil seeds, such as the Brassica seeds, cotton seeds, soybean, safflower, sunflower, or the like; grain seeds, e.g. wheat, barley, rice, clover, corn, or the like.

Identifying useful transcriptional initiation regions may be achieved in a number of ways. Where the seed protein has been or is isolated, it may be partially sequenced, so that a probe may be designed for identifying messenger RNA specific for seed. To further enhance the concentration of the messenger RNA specifically associated with seed, cDNA may be prepared and the cDNA subtracted with messenger RNA or cDNA from non-seed associated cells. The residual cDNA may then be used for probing the genome for complementary sequences, using an appropriate library prepared from plant cells. Sequences which hybridize to the cDNA may then be isolated, manipulated, and the 5'-untranslated region associated with the coding region isolated and used in expression constructs to identify the transcriptional activity of the 5'-untranslated region.

In some instances, the research effort may be further shortened by employing a probe directly for screening a genomic library and identifying sequences which hybridize to the probe. The sequences will be manipulated as described above to identify 5'-untranslated region.

The expression constructs which are prepared employing the 5'-untranslated regions may be transformed into plant cells as described previously for determination of their ability to function with a heterologous structural gene (other than the wild-type open reading frame associated with the 5'-untranslated region) and the seed specificity. In this manner, specific sequences may be identified for use with sequences for seed specific transcription. Expression cassettes of particular interest include transcriptional initiation regions from napin genes, particularly Brassica napin genes, more particularly Brassica napus or Brassica campestris genes, regulating structural genes associated with lipid production, particularly fatty acid production, including acyl carrier proteins, which may be endogenous or exogenous to the particular plant, such as spinach acyl carrier protein, Brassica acyl carrier protein, acyl carrier protein, either napus or campestris, Cuphea acyl carrier protein,, acetyl transacylase, malonyl transacylase, β-ketoacyl synthases I and II, thioesterase, particularly thioesterase II, from plant, mammalian, or bacterial sources, for example rat thioesterase II, acyl ACP, or phospholipid acyl desaturases.

The following examples are offered by way of illustration and not by way of limitation.

### EXPERIMENTAL

### Materials and Methods

### Cloning Vectors

Cloning vectors used include the pUC vectors, pUC8 and pUC9 (Vieira and Messing, Gene (1982) 19:259-268); pUC18 and pUC19 (Norrander et al., Gene (1983) 26:101-106; Yanisch-Perron et al., Gene - (1985) 33:103-119), and analogous vectors exchanging chloramphenicol resistance (CAM) as a marker for the ampicillin resistance of the pUC plasmids described above (pUC-CAM [pUC12-Cm, pUC13-Cm] Buckley, D., Ph.D. Thesis, U.C.S.D., CA 1985). The multiple cloning sites of pUC18 and pUC19 vectors were exchanged with those of pUC-CAM to create pCGN565 and pCGN566 which are CAM resistant. Also used were pUC118 and pUC119, which are respectively, pUC18 and pUC19 with the intergenic region of M13, from an HgiAI site at 5465 to the AhaIII site at 5941, inserted at the NdeI site of pUC. (Available from Vieira J. and Messing, J. Waksman Institute, Rutgers University, Rutgers, N.J.)

### Materials

Terminal deoxynucleotide transferase (TDT), RNaseH, E. coli DNA polymerase, T4 kinase, and restriction enzymes were obtained from Bethesda Research Laboratories; E. coli DNA ligase was obtained from New England Biolabs; reverse transcriptase was obtained from Life Sciences, Inc.; isotopes were obtained from Amersham; X-gal was obtained from Bachem, Inc. Torrance, CA.

### Example I

### Construction of a Napin Promoter

There are 298 nuclotides upstream of the ATG start codon of the napin gene on the pgN1 clone, a 3.3 kb EcoRI fragment of B. napus genomic DNA containing a napin gene cloned into pUC8 (available from Marti Crouch, University of Indiana). pgN1 DNA was digested with EcoRI and SstI and ligated to EcoRI/SstI digested pCGN706. (pCGN706 is an XhoI/PstI fragment containing 3' and polyadenylation sequences of another napin cDNA clone pN2 (Crouch et al., 1983 supra) cloned in pCGN566 at the SaII and PstI sites.) The resulting clone pCGN707 was digested with SaII and treated with the enzyme BaI31 to remove some of the coding region of the napin gene. The resulting resected DNA was digested with SmaI after the BaI31 treatment and religated. One of the clones, pCGN713, selected by size, was subcloned by EcoRI and BamHI digestion into both EcoRI/BamHI digested pEMBL18 (Dente et al., Nucleic Acids Res. (1983) 11:1645-1655) and pUC118 to give E418 and E4118 respectively. The extent of BaI31 digestion was conformed by Sanger dideoxy sequencing of E418 template. The BaI31 deletion of the promoter region extended only to 57 nucleotides downstream of the start codon, thus containing the 5' end of the napin coding sequence and about 300 bp of the 5' non-coding region. E4118 was tailored to delete all of the coding region of napin including the ATG start codon by in vitro mutagenesis by the method of Zoller and Smith (Nucleic Acids Res. (1982) 10:6487-6500) using an oligonucleotide primer 5'-GATGTTTTGTATGTGGGCCCCTAGGAGATC-3'.Screening for the appropriate mutant was done by two transformations into E. coli strain JM83 (Messing J., In: Recombinant DNA Technical Bulletin, NIH Publication No. 79-99, 2 No. 2, 1979, pp 43-48) and Smal digestion of putative transformants. The resulting napin promoter clone is pCGN778 and contains 298 nucleotides from the EcoRI site of pgN1 to the A nucleotide just before the ATG start codon of napin. The promoter region was subcloned into a chloramphenicol resistant background by digestion with EcoRI and BamHI and ligation to EcoRI/BamHI digested pCGN565 to give pCGN779c.

### Extension of the Napin Promoter Clone

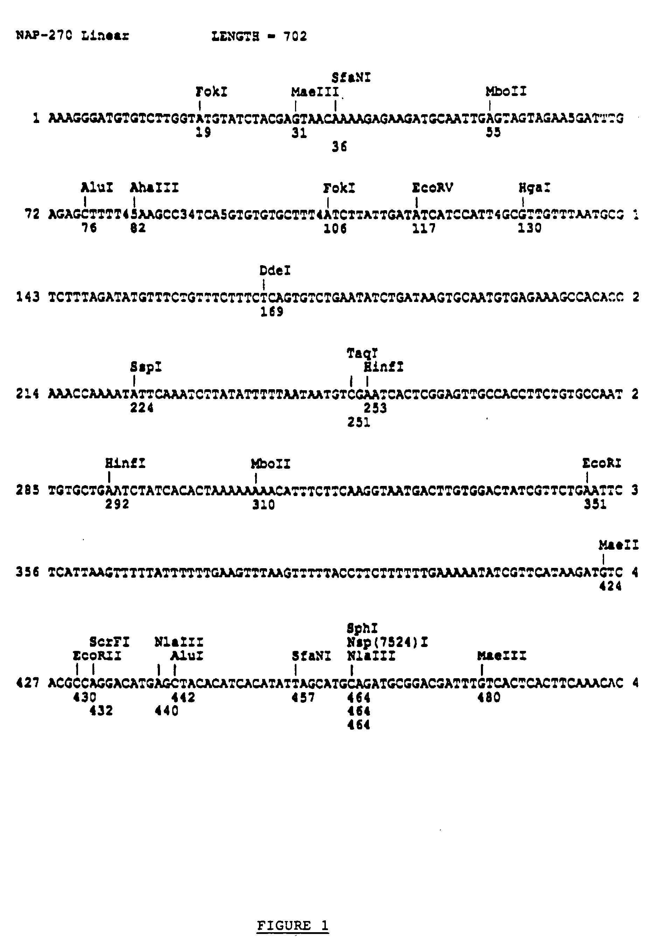

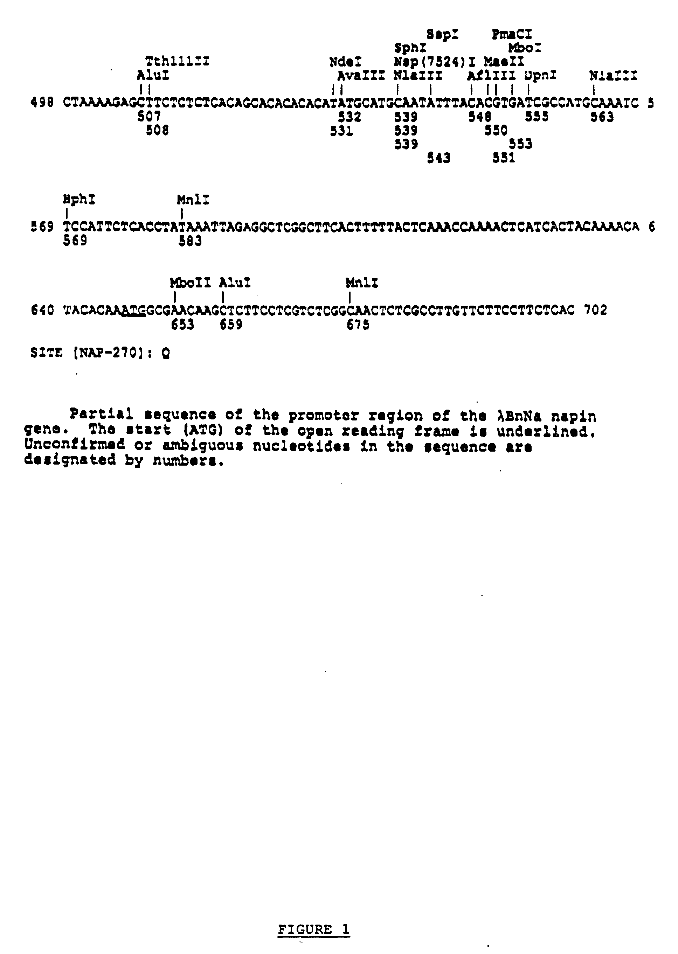

pCGN779c contains only 298 nucleotides of potential 5'-regulatory sequence. The napin promoter was extended with a 1.8 kb fragment found upstream of the 5'-EcoRI site on the original λBnNa clone. The ~3.5 kb XhoI fragment of λBnNa (available from M. Crouch), which includes the napin region, was subcloned into Sall-digested pUC119 to give pCGN930. A HindIII site close to a 5' XhoI site was used to subclone the HindIII/EcoRI fragment of pCGN930 into HindIII/EcoRI digested Bluescript + (Vector Cloning Systems, San Diego, CA) to give pCGN942. An extended napin promoter was made by ligating pCGN779c digested with EcoRI and PstI and pCGN942 digested with EcoRI and PstI to make pCGN943. This promoter contains ~2.1 kb of sequence upstream of the original ATG of the napin gene contained on λBnNa. A partial sequence of the promoter region is shown in Figure 1.

### Napin Cassettes

The extended napin promoter and a napin 3'-regulatory region is combined to make a napin cassette for expressing genes seed-specifically. The napin 3-region used is from the plasmid pCGN1924 containing the XhoI/EcoRI fragment from pgN1 (XhoI site is located 18 nucleotides from the stop codon of the napin gene) subcloned into EcoRI/SaII digested pCGN565. HindIII/PstI digested pCGN943 and pCGN1924 are ligated to make the napin cassette pCGN744, with unique cloning sites SmaI, SaII, and PstI for inserting genes.

### Construction of cDNA Library from Spinach Leaves

Total RNA was extracted from young spinach leaves in 4M guanidine thiocyanate buffer as described by Facciotti et al. (Biotechnology (1985) 3:241-246). Total RNA was subjected to oligo(dT)-cellulose column chromatography two times to yield poly(A)⁺ RNA as described by Maniatis et al., (1982) Molecular Cloning: A Laboratory Manual, Cold Spring Harbor Laboratory, New York. A cDNA library was constructed in pUC13-Cm aaccording to the method of Gubler and Hoffman, (Gene (1983) 25:263-269) with slight modifications. RNasin was omitted in the synthesis of first strand cDNA as it interfered with second strand synthesis if not completely removed, and dCTP was used to tail the vector DNA and dGTP to tail double-stranded cDNA instead of the reverse as described in the paper. The annealed cDNA was transformed to competent E. coli JM83 (Messing (1979) supra) cells according to Hanahan (J. Mol. Biol. (1983) 166:557-580) and spread onto LB agar plates (Miller (1972) Experiments in Molecular Genetics. Cold Spring Harbor Laboratory, Cold Spring Harbor, New York) containing 50 µg/ml chloramphenicol and 0.005% X-Gal.

### Identification of Spinach ACP-I cDNA

A total of approximately 8000 cDNA clones were screened by performing Southern blots (Southern, J. Mol. Biol. (1975) 98:503) and dot blot (described below) hybridizations with clone analysis DNA from 40 pools representing 200 cDNA clones each (see below). A 5' end labeled synthetic oligonucleotide (ACPP4) that is at least 66% homologous with a 16 amino acid region of spinach ACP-I (5'-GATGTCTTGAGCCTTGTCCTCATCCACATTGATACCAAACTCCTCCTC-3') is the complement to a DNA sequence that could encode the 16 amino acid peptide glu-glu-glu-phe-gly-ile-asn-val-asp-glu-asp-lys-ala-gln-asp-ile, residues 49-64 of spinach ACP-I (Kuo and Ohlrogge, Arch. Biochem. Biophys. (1984) 234:290-296) and was used for an ACP probe.

Clone analysis DNA for Southern and dot blot hybridizations was prepared as follows. Transformants were transferred from agar plates to LB containing 50 µg/ml chloramphenicol in groups of ten clones per 10 ml media. Cultures were incubated overnight in a 37°C shaking incubator and then diluted with an equal volume of media and allowed to grow for 5 more hours. Pools of 200 cDNA clones each were obtained by mixing contents of 20 samples. DNA was extracted from these cells as described by Birnboim and Doly (Nucleic Acids Res. (1979) 7:1513-1523). DNA was purified to enable digestion with restriction enzymes by extractions with phenol and chloroform followed by ethanol precipitation. DNA was resuspended in sterile, distilled water and 1 µg of each of the 40 pooled DNA samples was digested with EcoRI and HindIII and electrophoresed through 0.7% agarose gels. DNA was transferred to nitrocellulose filters following the blot hybridization technique of Southern.

ACPP4 was 5' end-labeled using γ-³²P dATP and T4 kinase according to the manufacturer's specifications. Nitrocellulose filters from Southern blot transfer of clone analysis DNA were hybridized (24 hours, 42 °C) and washed according to Berent et al. (BioTechniques (1985) 3:208-220). Dot blots of the same set of DNA pools were prepared by applying 1 µg of each DNA pool to nylon membrane filters in 0.5 M NaOH. These blots were hybridized with the probe for 24 hours at 42°C in 50% formamide/1% SDS/1 M NaCL, and washed at room temperature in 2X SSC/0.1% SDS (1X SSC = 0.15M NaCI; 0.015M Na citrate; SDS-sodium dodecylsulfate). DNA from the pool which was hybridized by the ACPP4 oligoprobe was transformed to JM83 cells and plated as above to yield individual transformants. Dot blots of these individual cDNA clones were prepared by applying DNA to nitrocellulose filters which were hybridized with the ACPP4 oligonucleotide probe and analyzed using the same conditions as for the Southern blots of pooled DNA samples.

### Nucleotide Sequence Analysis

The positive clone, pCGN1SOL, was analyzed by digestion with restriction enzymes and the following partial map was obtained.

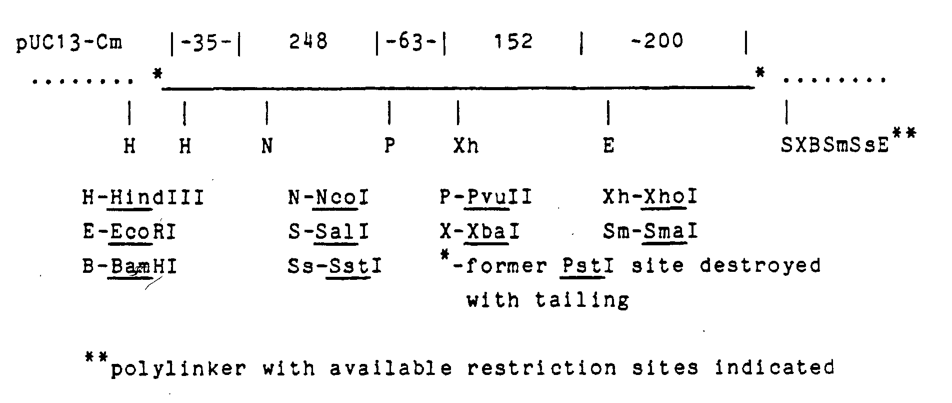

The cDNA clone was subcloned into pUC118 and pUC119 using standard laboratory techniques of restriction, ligation, transformation, and analysis (Maniatis et al., (1982) supra). Single-stranded DNA template was prepared and DNA sequence was determined using the Sanger dideoxy technique (Sanger et al., (1977) Proc. Nat. Acad. Sci. USA 74:5463-5467). Sequence analysis was performed using a software package from Intelli-Genetics, Inc.

pCGNISOL contains an (approximately) 700 bp cDNA insert including a stretch of A residues at the 3' terminus which represents the poly(A) tail of the mRNA. An ATG codon at position 61 is presumed to encode the MET translation initiation codon. This codon is the start of a 411 nucleotide open reading frame, of which, nucleotides 229-471 encode a protein whose amino acid sequence corresponds almost perfectly with the published amino acid sequence of ACP-I of Kuo and Ohlrogge supra as described previously. In addition to mature protein, the pCGN1SOL also encodes a 56 residue transit peptide sequence, as might be expected for a nuclear-encoded chloroplast protein.

### Napin - ACP Construct

pCGN796 was constructed by ligating pCGN1SOL digested with HindIII/BamHI, pUC8 digested with HindIII and BamHI and pUC118 digested with BamHI. The ACP gene from pCGN796 was transferred into a chloramphenicol background by digestion with BamHI and ligation with BamHI digested pCGN565. The resulting pCGN1902 was digested with EcoRI and SmaI and ligated to EcoRI/SmaI digested pUC118 to give pCGN1920. The ACP gene in pCGN1920 was digested at the NcoI site, filled in by treatment with the Klenow fragment, digested with SmaI and religated to form pCGN1919. This eliminated the 5'-coding sequences from the ACP gene and regenerated the ATG. This ACP gene was flanked with PstI sites by digesting pCGN1919 with EcoRI, filling in the site with the Klenow fragment and ligating a PstI linker. This clone is called pCGN945.

The ACP gene of pCGN945 was moved as a BamHI/PstI fragment to pUC118 digested with BamHI and PstI to create pCGN945a so that a SmaI site (provided by the pUC118) would be at the 5'-end of the ACP sequences to facilitate cloning into the napin cassette pCGN944. pCGN945a digested with SmaI and PstI was ligated to pCGN944 digested with Smal and PstI to produce the napin ACP cassette pCGN946. The napin ACP cassette was then transferred into the binary vector pCGN783 by cloning from the HindIII site to produce pCGN948.

### Construction of the Binary Vector pCGN783

pCGN783 is a binary plasmid containing the left and right T-DNA borders of A. tumefaciens (Barker et al., Plant Mol. Biol. (1983) 2:335-350); the gentamicin resistance gene of pPH1JI (Hirsch et al., Plasmid - (1984), 12:139-141) the 35S promoter of cauliflower mosaic virus (CaMV) (Gardner et al., Nucleic Acids Res. (1981) 9:2871-2890), the kanamycin resistance gene of Tn5 (Jorgenson et al., infra and Wolff et al., ibid (1985) 13:355-367) and the 3' region from transcript 7 of pTiA6 (Barker et al., supra (1983)).

To obtain the gentamicin resistance marker, the gentamicin resistance gene was isolated from a 3.1 kb EcoRI-PstI fragment of pPHIJ1 and cloned into pUC9 yielding pCGN549. The HindIII-BamHI fragment containing the gentamicin resistance gene was substituted for the HindIII-BgIII fragment of pCGN587 creating pCGN594.

pCGN587 was prepared as follows: The HindIII-SmaI fragment of Tn5 containing the entire structural gene for APHII (Jorgenson et al., Mol. Gen. Genet. (1979) 177:65) was cloned into pUC8 (Vieira and Messing, Gene (1982) 19:259), converting the fragment into a HindIII-EcoRI fragment, since there is an EcoRI site immediately adjacent to the SmaI site. The PstI-EcoRI fragment containing the 3'-portion of the APHII gene was then combined with an EcoRI-BamHI-SaII-PstI linker into the EcoRI site of pUC7 (pCGN546W). Since this construct does not confer kanamycin resistance, kanamycin resistance was obtained by inserting the BgIII-PstI fragment of the APHII gene into the BamHI-PstI site (pCGN546X). This procedure reassembles the APHII gene, so that Eco sites flank the gene. An ATG codon was upstream from and out of reading frame with the ATG initiation codon of APHII. The undesired ATG was avoided by inserting a Sau3A-PstI fragment from the 5'-end of APHII, which fragment lacks the superfluous ATG, into the BamHI-PstI site of pCGN546W to provide plasmid pCGN550.

The EcoRI fragment containing the APHII gene was then cloned into the unique EcoRI site of pCGN451, which contains an octopine synthase cassette for expression, to provide pCGN552 (1ATG).

pCGN451 includes an octopine cassette which contains about 1556 bp of the 5' non-coding region fused via an EcoRI linker to the 3' non-coding region of the octopine synthase gene of pTiA6. The pTi coordinates are 11,207 to 12,823 for the 3' region and 13,643 to 15,208 for the 5' region as defined by Barker et al., Plant Mol. Biol. (1983) 2:325.

The 5' fragment was obtained as follows. A small subcloned fragment containing the 5' end of the coding region, as a BamHI-EcoRI fragment was cloned in pBR322 as plasmid pCGN407. The BamHI-EcoRI fragment has an XmnI site in the coding region, while pBR322 has two XmnI sites. pCGN407 was digested with XmnI, resected with BaI31 nuclease and EcoRI linkers added to the fragments. After EcoRI and BamHI digestion, the fragments were size fractionated, the fractions cloned and sequenced. In one case, the entire coding region and 10 bp of the 5' non-translated sequences had been removed leaving the 5' non-translated region, the mRNA cap site and 16 bp of the 5' non-translated region (to a BamHI site) intact. This small fragment was obtained by size fractionation on a 7% acrylamide gel and fragments approximately 130 bp long eluted.

This size fractionated DNA was ligated into M13mp9 and several clones sequenced and the sequence compared to the known sequence of the octopine synthase gene. The M13 construct was designated p14, which plasmid was digested with BamHI and EcoRI to provide the small fragment which was ligated to a XhoI to BamHI fragment containing upstream 5 sequences from pTiA6 (Garfinkel and Nester, J. Bacteriol. (1980) 144:732) and to an EcoRI to XhoI fragment containing the 3' sequences.

The resulting Xhol fragment was cloned into the Xhol site of a pUC8 derivative, designated pCGN426. This plasmid differs from pUC8 by having the sole EcoRI site filled in with DNA polymerase I, and having lost the PstI and HindIII site by nuclease contamination of HincII restriction endonuclease, when a XhoI linker was inserted into the unique HincII site of pUC8. The resulting plasmid pCGN451 has a single EcoRI site for the insertion of protein coding sequences between the 5' non-coding region (which contains 1,550 bp of 5' non-transcribed sequence including the right border of the T-DNA, the mRNA cap site and 16 bp of 5' non-translated sequence) and the 3' region (which contains 267 bp of the coding region, the stop codon, 196 bp of 3' non-translated DNA, the polyA site and 1,153 bp of 3' non-transcribed sequence). pCGN451 also provides the right T-DNA border.

The resulting plasmid pCGN451 having the ocs 5' and the ocs 3' in the proper orientation was digested with EcoRI and the EcoRI fragment from pCGN551 containing the intact kanamycin resistance gene inserted into the EcoRI site to provide pCGN552 having the kanamycin resistance gene in the proper orientation.

This ocs/KAN gene was used to provide a selectable marker for the trans type binary vector pCGN587.

The 5' portion of the engineered octopine synthase promoter cassette consists of pTiA6 DNA from the XhoI at bp 15208-13644 (Barker's numbering), which also contains the T-DNA boundary sequence (border) implicated in T-DNA transfer. In the plasmid pCGN587, the ocs/KAN gene from pCGN552 provides a selectable marker as well as the right border. The left boundary region was first cloned in M13mp9 as a HindIII-SmaI piece (pCGN502) (base pairs 602-2213) and recloned as a KpnI-EcoRI fragment in pCGN565 to provide pCGN580. pCGN565 is a cloning vector based on pUC8-Cm, but containing pUC18 linkers. pCGN580 was linearized with BamHI and used to replace the smaller BgIII fragment of pVCK102 (Knauf and Nester, Plasmid (1982) 8:45), creating pCGN585. By replacing the smaller SaII fragment of pCGN585 with the XhoI fragment from pCGN552 containing the ocs/KAN gene, pCGN587 was obtained.

The pCGN594 HindIII-BamHI region, which contains an 5'-ocs-kanamycin-ocs-3' (ocs is octopine synthase with 5' designating the promoter region and 3' the terminator region, see U.S. application serial no. 775,923, filed September 13, 1985) fragment was replaced with the HindIII-BamHI polylinker region from pUC18.

pCGN566 contains the EcoRI-HindIII linker of pUC18 inserted into the EcoRI-HindIII sites of pUC13-Cm.

The HindIII-BgIII fragment of pNW31C-8,29-1 (Thomashow et al., Cell (1980) 19:729) containing ORF1 and -2 of pTiA6 was subcloned into the HindIII-BamHI sites of pCGN566 producing pCGN703.

The Sau3A fragment of pCGN703 containing the 3' region of transcript 7 (corresponding to bases 2396-2920 of pTiA6 (Barker et al., (1983) supra) was sub-cloned into the BamHI site of pUC18 producing pCGN709. The EcoRI-SmaI polylinker region of pCGN709 was substituted with the EcoRI-SmaI fragment of pCGN587, which contains the kanamycin resistance gene (APH3-II) producing pCGN726.

The EcoRI-SaII fragment of pCGN726 plus the BgIII-EcoRI fragment of pCGN734 were inserted into the BamHI-SaII site of pUC8-Cm producing pCGN738. pCGN726c is derived from pCGN738 by deleting the 900 bp EcoRI-EcoRI fragment.

To construct pCGN167, the AluI fragment of CaMV (bp 7144-7735) (Gardner et al., Nucl. Acid Res. - (1981) 9:2871-2888) was obtained by digestion with AluI and cloned into the HincII site of M13mp7 (Messing et al., Nucl. Acids Res. (1981) 9:309-321) to create C614. An EcoRI digest of C614 produced the EcoRI fragment from C614 containing the 35S promoter which was cloned into the EcoRI site-of pUC8 (Vieira and Messing, Gene (1982) 19:259) to produce pCGN146.

To trim the promoter region, the BgIII site (bp 7670) was treated with BgIII and resected with BaI31 and subsequently a BgIII linker was attached to the BaI31 treated DNA to produce pCGN147.

pCGN148a containing a promoter region, selectable marker (KAN with 2 ATG's) and 3' region, was prepared by digesting pCGN528 with BgIII and inserting the BamHI-BgIII promoter fragment from pCGN147. This fragment was cloned into the BgIII site of pCGN528 so that the BgIII site was proximal to the kanamycin gene of pCGN528.

The shuttle vector used for this construct, pCGN528, was made as follows. pCGN525 was made by digesting a plasmid containing Tn5 which harbors a kanamycin gene (Jorgenson et al., Mol. Gen. Genet. 1979) 177:65) with HindIII-BamHI and inserting the HindIII-BamHI fragment containing the kanamycin gene into the HindIII-BamHI sites in the tetracycline gene of pACYC184 (Chang and Cohen, J. Bacteriol. (1978) 134:1141-1156). pCGN526 was made by inserting the BamHI fragment 19 of pTiA6 (Thomashow et al., Cell (1980) 19:729-739), modified with XhoI linkers inserted into the SmaI site, into the BamHI site of pCGN525. pCGN528 was obtained by deleting the small XhoI fragment from pCGN526 by digesting with XhoI and religating.

pCGN149a was made by cloning the BamHI-kanamycin gene fragment from pMB9KanXXI into the BamHI site of pCGN148a.

pMB9KanXXI is a pUC4K variant (Vieira and Messing, Gene (1982) 19:259-268) which has the XhoI site missing but contains a functional kanamycin gene from Tn903 to allow for efficient selection in Agrobacterium.

pCGN149a was digested with BgIII and SphI. This small BgIII-SphI fragment of pCGN149a was replaced with the BamHI-SphI fragment from MI (see below) isolated by digestion with BamHI and SphI. This produces pCGN167, a construct containing a full length CaMV promoter, 1ATG-kanamycin gene, 3' end and the bacterial Tn903-type kanamycin gene. MI is an EcoRI fragment from pCGN546X (see construction of pCGN587) and was cloned into the EcoRI cloning site of M13mp9 in such a way that the PstI site in the 1ATG-kanamycin gene was proximal to the polylinker region of M13mp9.

The HindIII-BamHI fragment in the pCGN167 containing the CaMV-35S promoter, 1ATG-kanamycin gene and the BamHI-fragment 19 of pTiA6 was cloned into the BamHI-HindIII sites of pUC19 creating pCGN976. The 35S promoter and 3' region from transcript 7 was developed by inserting a 0.7 kb HindIII-EcoRI fragment of pCGN976 (35S promoter) and the 0.5 kb EcoRI-SauI fragment of pCGN709 (transcript 7:3) into the HindIII-SaII sites of pCGN566 creating pCGN766c.

The 0.7 kb HindIII-EcoRI fragment of pCGN766c (CaMV-35S promoter) was ligated to the 1.5 kb EcoRI-SaII fragment in pCGN726c (1ATG-KAN 3' region) followed by insertion into the HindIII-SaII sites of pUC119 to produce pCGN778. The 2.2 kb region of pCGN778, HindIII-SaII fragment containing the CaMV-355 promoter and 1ATG-KAN-3' region was used to replace the HindIII-SaII linker region of pCGN739 to produce pCGN783.

### Transfer of the Binary Vector pCGN948 into Agrobacterium

pCGN948 was introduced into Agrobacterium tumefaciens EHA101 (Hood et al., J. Bacteriol. (1986) 168:1291-1301) by transformation. An overnight 2 ml culture of EHA101 was grown in MG/L broth at 30°C. 0.5 ml was inoculated into 100 ml of MG/L broth (Garfinkel and Nester, J. Bacteriol. (1980) 144:732-743) and grown in a shaking incubator for 5 h at 30°C. The cells were pelleted by centrifugation at 7K, resuspended in 1 ml of MG/L broth and placed on ice. Approximately, 1 µg of pCGN948 DNA was placed in 100 µl of MG/L broth to which 200 µl of the EHA101 suspension was added; the tube containing the DNA-cell mix was immediately placed into a dry ice/ethanol bath for 5 minutes. The tube was quick thawed by 5 minutes in 37°C water bath followed by 2 h of shaking at 30°C after adding 1 ml of fresh MG/L medium. The cells were pelleted and spread onto MG/L plates (1.5% agar) containing 100 mg/l gentamicin. Plasmid DNA was isolated from individual gentamicin-resistant colonies, transformed back into E. coli, and characterized by restriction enzyme analysis to verify that the gentamicin-resistant EHA101 contained intact copies of pCGN948. Single colonies are picked and purified by two more streakings on MG/L plates containing 100 mg/I gentamicin.

### Transformation and Regeneration of B. Napus

Seeds of Brassica napus cv Westar were soaked in 95% ethanol for 4 minutes. They were sterilized in 1% solution of sodium hypochlorite with 50 µl of "Tween 20" surfactant per 100 ml sterilent solution. After soaking for 45 minutes, seeds were rinsed 4 times with sterile distilled water. They were planted in sterile plastic boxes 7 cm wide, 7 cm long, and 10 cm high (Magenta) containing 50 ml of 1/10th concentration of MS (Murashige minimal organics medium, Gibco) with added pyridoxine (500 µg/l), nicotinic acid (50 µg/l), glycine (200 µg/l) and solidified with 0.6% agar. The seeds germinated and were grown at 22°C in a 16h-8h light-dark cycle with light intensity approximately 65 µEm⁻²s⁻¹. After 5 days the seedlings were taken under sterile conditions and the hypocotyls excised and cut into pieces of about 4 mm in length. The hypocotyl segments were placed on a feeder plate or without the feeder layer on top of a filter paper on the soldified B5 0/1/1 or B5 0/1/0 medium. B5 0/1/0 medium contains B5 salts and vitamins (Gamborg, Miller and Ojima, Experimental Cell Res. (1968) 50:151-158), 3% sucrose, 2,4-dichlorophenoxyacetic acid (1.0 mg/l), pH adjusted to 5.8, and the medium is solidifed with 0.6% Phytagar; B5 0/1/1 is the same with the addition of 1.0 mg/l kinetin. Feeder plates were prepared 24 hours in advance by pipetting 1.0 ml of a stationary phase tobacco suspension culture (maintained as described in Fillatti et al., Molecular General Genetics (1987) 206:192-199) onto B5 0/1/0 or B5 0/1/1 medium. Hypocotyl segments were cut and placed on feeder plates 24 hours prior to Agrobacterium treatment.

Agrobacterium tumefaciens (strain EHA101 x 948) was prepared by incubating a single colony of Agrobacterium in MG/L broth at 30°C. Bacteria were harvested 16 hours later and dilutions of 10⁸ bacteria per ml were prepared in MG/L broth. Hypocotyl segments were inoculated with bacteria by placing the segments in an Agrobacterium suspension and allowing them to sit for 30-60 minutes, then removing and transferring to Petri plates containing B5 0/1/1 or 0/1/0 medium (0/1/1 intends 1 mg/l 2,4-D and 1 mg/l kinetin and 0/1/0 intends no kinetin). The plates were incubated in low light at 22°C. The co-incubation of bacteria with the hypocotyl segments took place for 24-48 hours. The hypocotyl segments were removed and placed on B5 0/1/1 or 0/1/0 containing 500 mg/l carbenicillin (kanamycin sulfate at 10, 25, or 50 mg/l was sometimes added at this time) for 7 days in continuous light (approximately 65 µEm⁻²S⁻¹) at 22°C. The segments were transferred to B5 salts medium containing 1% sucrose, 3 mg/l benzylamino purine (BAP) and 1 mg/l zeatin. This was supplemented with 500 mg/l carbenicillin, 10, 25, or 50 mg/l kanamycin sulfate, and solidified with 0.6% Phytagar (Gibco). Thereafter, explants were transferred to fresh medium every two weeks.

After one month green shoots developed from green calli which were selected on media containing kanamyicin. Shoots continued to develop for three months. The shoots were cut from the calli when they were at least 1 cm high and placed on B5 medium with 1% sucrose, no added growth substances, 300 mg/l carbencillin, and solidified with 0.6% phytagar. The shoots continued to grow and several leaves were removed to test for neomycin phosphotransferase II (NPTII) activity. Shoots which were positive for NPTII activity were placed in Magenta boxes containing B5 0/1/1 medium with 1% sucrose, 2 mg/l indolebutyric acid, 200 mg/l carbenicillin, and solidified with 0.6% Phytagar. After a few weeks the shoots developed roots and were transferred to soil. The plants were grown in a growth chamber at 22°C in a 16-8 hours light-dark cycle with light intensity 220 µEm⁻²s⁻¹ and after several weeks were transferred to the greenhouse.

### Southern Data

Regenerated B. napus plants from cocultivations of Agrobacterium tumefaciens EHA101 containing pCGN948 and B. napus hypocotyls were examined for proper integration and embryo-specific expression of the spinach leaf ACP gene. Southern analysis was performed using DNA isolated from leaves of regenerated plants by the method of Dellaporta et al., (Plant Mol. Biol. Rep. (1983) 1:19-21) and purified once by banding in CsCI. DNA (10 µg) was digested with the restriction enzyme EcoRI, electrophoresed on a 0.7% agarose gel and blotted to nitrocellulose (see Maniatis et al., (1982) supra.). Blots were probed with pCGN945 DNA containing 1.8 kb of the spinach ACP sequence or with the EcoRI/ HindIII fragment isolated from pCGN936c (made by transferring the HindlII/EcoRI fragment of pCGN930 into pCGN566) containing the napin 5' sequences labeled with ³²P-dCTP by nick translation (described by the manufacturer, BRL Nick Translation Reagent Kit, Bethesda Research Laboratories, Gaithersburg, MD). Blots were prehybridized and hybridized in 50% formamide, 10x Denhardt's 5xSSC, 0.1% SDS, 5 mM EDTA, 100 µg/ml calf thymus DNA and 10% dextran sulfate (hybridization only) at 42°C. (Reagents described in Maniatis et al., (1982) supra.) Washes were in 1xSSC, 0.1% SDS, 30 min and twice in 0.1xSSC, 0.1% SDS at 55°C.

Autoradiograms showed two bands of approximately 3.3 and 3.2 kb hybridized in the EcoRI digests of DNA from four plants when probed with the ACP gene (pCGN945) indicating proper integration of the spinach leaf ACP construct in the plant genome since 3.3 and 3.2 kb EcoRI fragments are present in the T-DNA region of pCGN948. The gene construct was present in single or multiple loci in the different plants as judged by the number of plant DNA-construct DNA border fragments detected when probed with the napin 5' sequences.

### Northern Data

Expression of the integrated spinach leaf ACP gene from the napin promoter was detected by Northern analysis in seeds but not leaves of one of the transformed plants shown to contain the construct DNA. Developing seeds were collected from the transformed plant 21 days post-anthesis. Embryos were dissected from the seeds and frozen in liquid nitrogen. Total RNA was isolated from the seed embryos and from leaves of the transformed plant by the method of Crouch et al., 1983, supra, electrophoresed on formaldehyde-containing 1.5% agarose gels as described (Shewmaker et al., Virology (1985) 140:281-288) and blotted to nitrocellulose (Thomas, Proc. Natl. Acad. Sci. USA (1980) 77:5201-5205). Blots were prehybridized, hybridized, and washed as described above. The probe was an isolated PstI/BamHI fragment from pCGN945 containing only spinach leaf ACP sequences labeled by nick translation.

An RNA band of ∼0.8 kb was detected in embryos but not leaves of the transformed plant indicating seed-specific expression of the spinach leaf ACP gene.

### Example II

### Construction of B. Campestris Napin Promoter Cassette

A BgIII partial genomic library of B. campestris DNA was made in the lambda vector Charon 35 using established protocols (Maniatis et al., 1982, supra). The titer of the amplified library was ∼1.2 x 10⁹ phage/ml. Four hundred thousand recombinant bacteriophage were plated at a density of 10⁵ per 9 x 9 in. NZY plate (NZYM as described in Maniatis et al., 1982, supra) in NZY + 10 mM MgSO₄ + 0.9% agarose after adsorption to DH1 E. coli cells (Hanahan, Mol. Biol. (1983) 166:557) for 20 min at 37°C. Plates were incubated at 37°C for ∼13 hours, cooled at 4°C for 2.5 hours and the phage were lifted onto Gene Screen Plus (New England Nuclear) by laying precut filters over the plates for approximately 1 min and peeling them off. The adsorbed phage DNA was immobilized by floating the filter on 1.5 M NaCI, 0.5 M NaOH for 1 min., neutralizing in 1.5 M NaCI, 0.5 M Tris-HCl, pH 8.0 for 2 min and 2XSSC for 3 min. Filters were air dried until just damp, prehybridized and hybridized at 42 °C as described for Southern analysis. Filters were probed for napin-containing clones using an XhoI/SaII fragment of the cDNA clone BE5 which was isolated from the B. campestris seed cDNA library described using the probe pN1 (Crouch et al., 1983, supra). Three plaques were hybridized strongly on duplicate filters and were plaque purified as described (Maniatis et al., 1982, supra).

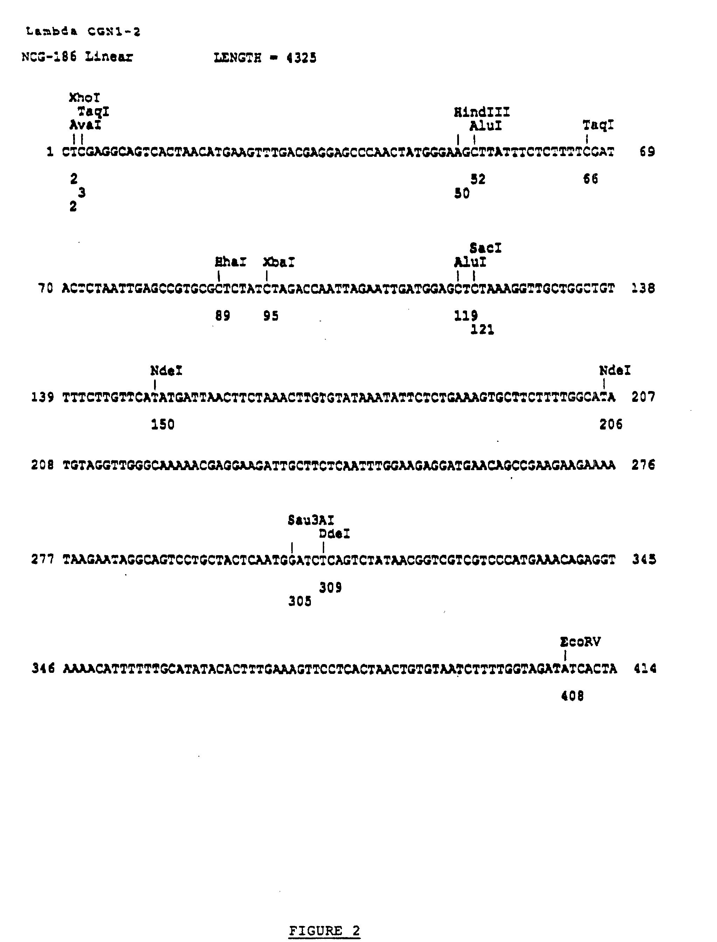

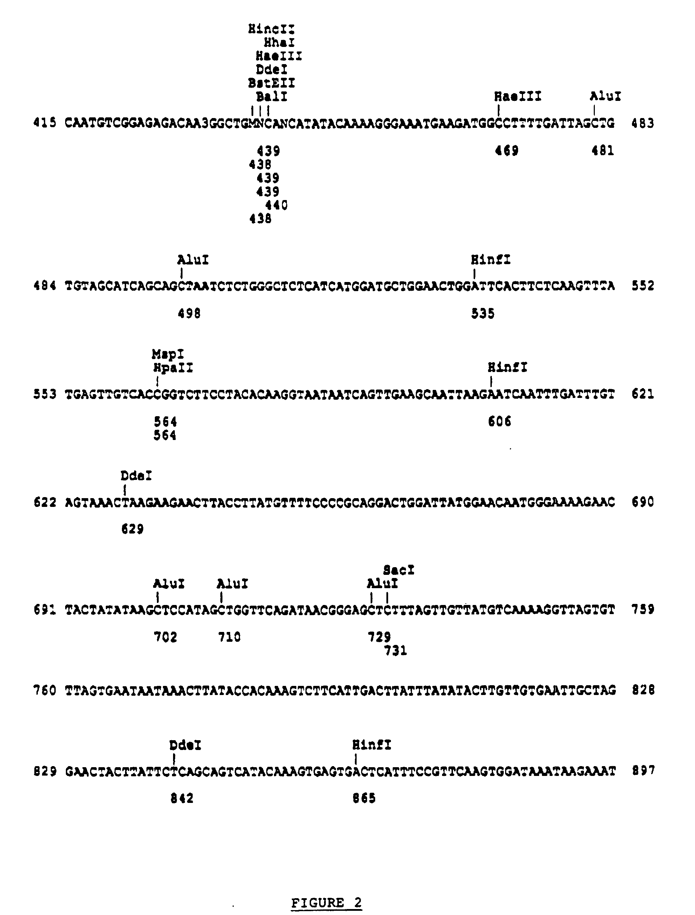

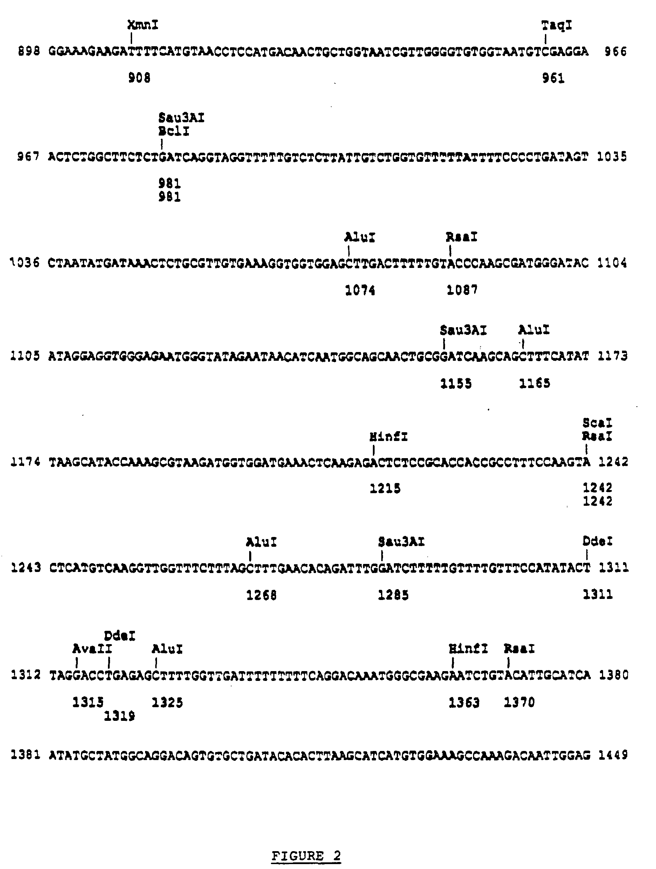

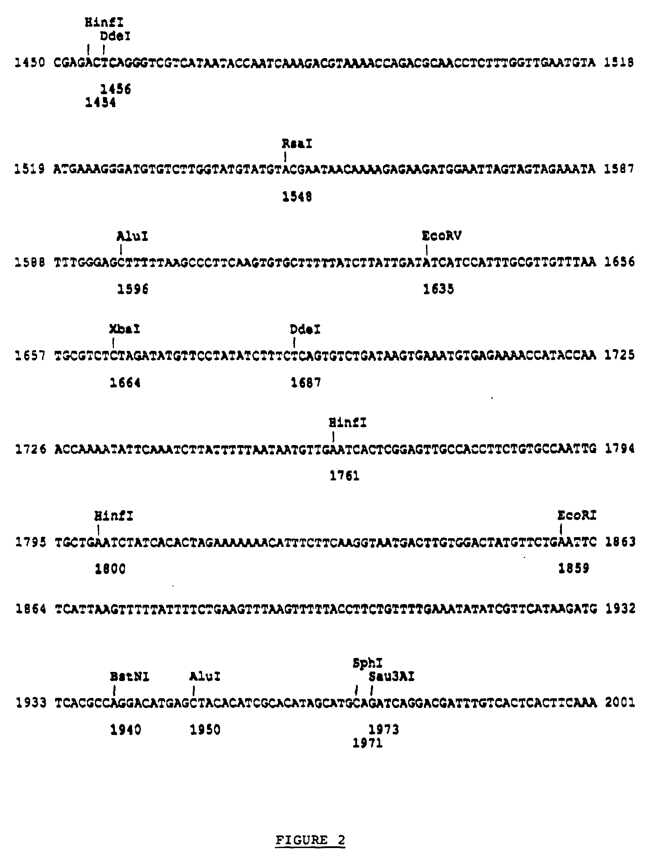

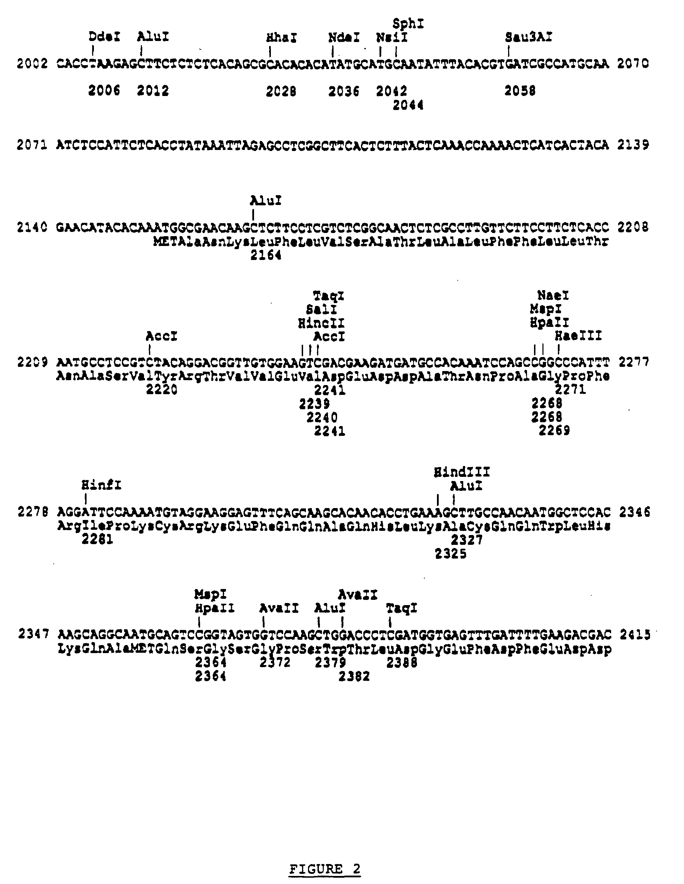

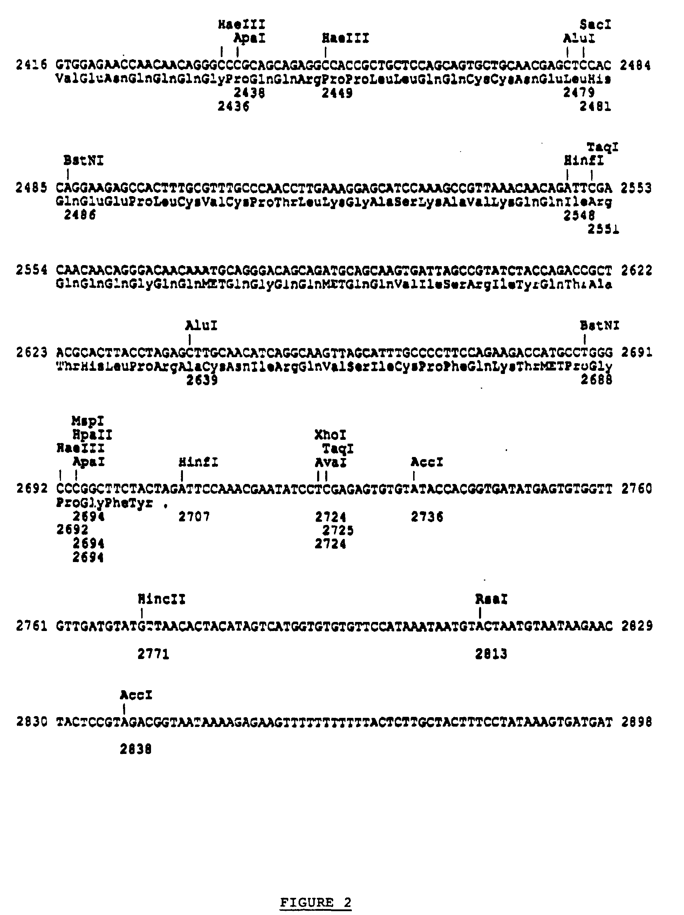

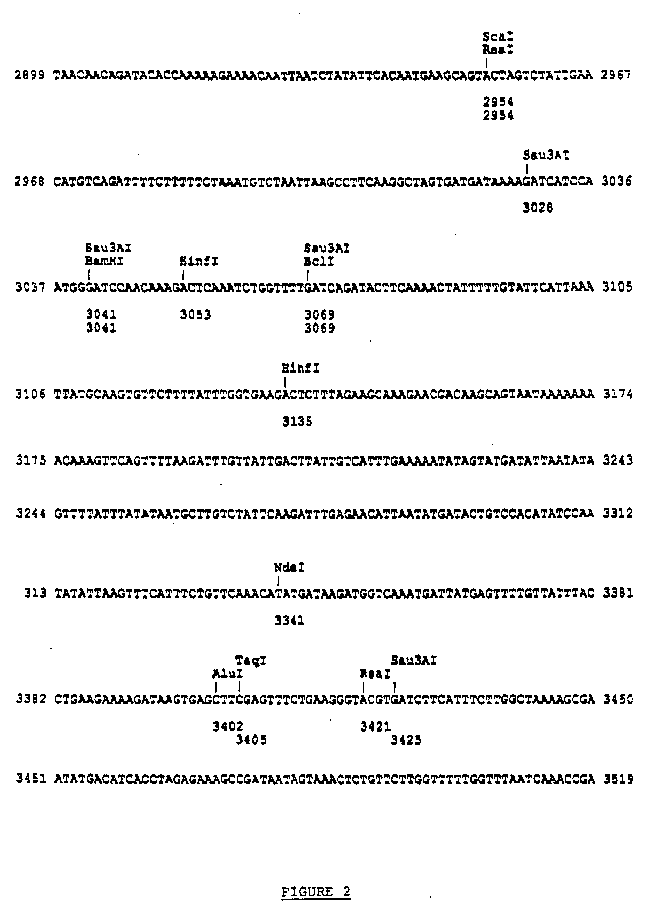

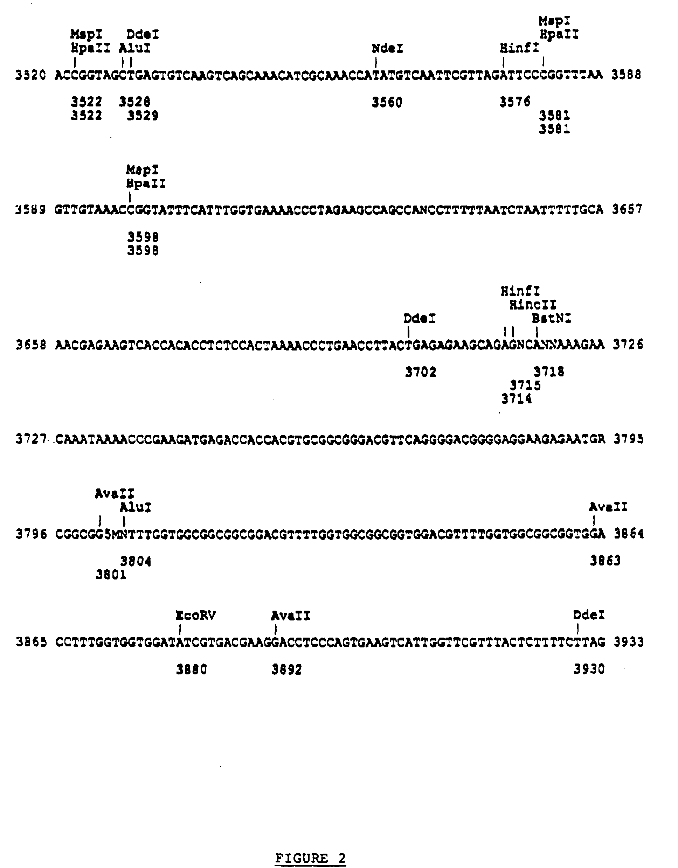

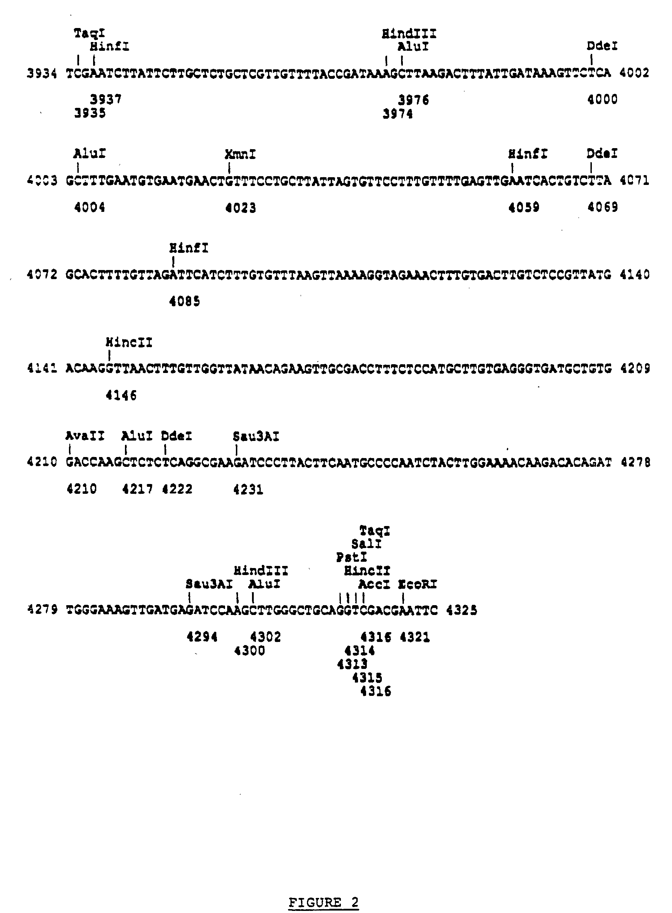

One of the clones named lambda CGN1-2 was restriction mapped and the napin gene was localized to overlapping 2.7 kb Xhol and 2.1 kb Sail restriction fragments. The two fragments were subcloned from lambda CGN1-2 DNA into pCGN789 (a pUC based vector the same as pUC119 with the normal polylinker replaced by the synthetic linker - 5' GGAATTCGTCGACAGATCTCTGCAGCTCGAGGGATCCAAGCTT 3' - (which represents the polylinker EcoRI, SaII, BgIII, PstI, XhoI, BamHI, HindIII). The identity of the subclones as napin was confirmed by sequencing. The entire coding region sequence as well as extensive 5' upstream and 3' downstream sequences were determined (Figure 2). The lambda CGN1-2 napin gene is that encoding the mRNA corresponding to the BE5 cDNA as determined by the exact match of their nucleotide sequences.

An expression cassette was constructed from the 5'-end and the 3'-end of the lambda CGN1-2 napin gene as follows in an analogous manner to the construction of pCGN944. The majority of the napin coding region of pCGN940 was deleted by digestion with SaII and religation to form pCGN1800. Single-stranded DNA from pCGN1800 was used in an in vitro mutagenesis reaction (Adelman et al., DNA (1983) 2:183-193) using the synthetic oligonucleotide 5' GCTTGTTCGCCATGGATATCTTCTGTATGTTC 3'. This oligonucleotide inserted an EcoRV and an Nco1 restriction site at the junction of the promoter region and the ATG start codon of the napin gene. An appropriate mutant was identified by hybridization to the oligonucleotide used for the mutagenesis and sequence analysis and named pCGN1801.

A 1.7 kb promoter fragment was subcloned from pCGN1801 by partial digestion with EcoRV and ligation to pCGN786 (a pCGN566 chloramphenicol based vector with the synthetic linker described above in place of the normal polylinker) cut with EcoRI and blunted by fill## resulting expression cassette, pCGN1803 contains 1.725 kb of napin promoter sequence, and 1.265 kb of napin 3' sequences with the unique cloning sites SaII, BgII, PstI, and XhoI in between. Any sequence that requires seed-specific transcription or expression in Brassica, i.e., a fatty acid gene could be inserted in this cassette in a manner analogous to that described for spinach leaf ACP and the B. napus napin cassette in Example I.

### Example III

Other seed-specific promoters may be isolated from genes encoding proteins involved in seed triacylglycerol synthesis, such as acyl carrier protein from Brassica seeds. Immature seed were collected from Brassica campestris cv. "R-500," a self-compatible variety of turnip rape. Whole seeds were collected at stages corresponding approximately to 14 to 28 days after flowering. RNA isolation and preparation of a cDNA bank was as described above for the isolation of a spinach ACP cDNA clone except that the vector used was pCGN565. To probe the cDNA bank, the oligonucleotide (5')-ACTTTCTCAACTGTCTCTGGTTTAGCAGC-(3') was synthesized using an Applied Biosystems DNA Synthesizer, model 380A, according to manufacturer's recommendations. This synthetic DNA molecule will hybridize at low stringencies to DNA or RNA sequences coding for the amino acid sequence (ala-ala-lys-pro-glu-thr-val-glu-lys-val). This amino acid sequence has been reported for ACP isolated from seeds of Brassica napus (Slabas et al., 7th International Symposium of the Structure and Function of Plant Lipids, University of California, Davis, CA, 1986); ACP from B. campestris seed is highly homologous. Approximately 2200 different cDNA clones were analyzed using a colony hybridization technique (Taub and Thompson, Anal. Biochem. (1982) 126:222-230) and hybridization conditions corresponding to Wood et al., (Proc. Natl. Acad. Sci. (1985) 82:1585-1588). DNA sequence analysis of two cDNA clones showing obvious hybridization to the oligonucleotide probe indicated that one, designated pCGN1Bcs, indeed coded for an ACP-precursor protein by the considerable homology of the encoded amino acid sequence with ACP proteins described from Brassica napus (Slabas et al., 1980 supra). Similarly to Example II, the ACP cDNA clone can be used to isolate a genomic clone from which an expression cassette can be fashioned in a manner directly analogous to the B. campestris napin cassette.

### Other Examples

Ninety-six clones from the 14-28 day post-anthesis B. campestris seed cDNA library (described in the previous example) were screened by dot blot hybridization of miniprep DNA on Gene Screen Plus nylon filters. Probes used were radioactively labeled first-strand synthesis cDNAs made from the day 14-28 post-anthesis mRNA or from B. campestris leaf mRNA. Clones which hybridized strongly to seed cDNA and little or not at all to leaf cDNA were catalogued. A number of clones were identified as representing the seed storage protein napin by cross-hybridization with an XhoI/SaII fragment of pNI (Crouch et al., 1983, supra), a B. campestris genomic clone as a source of an embryo-specific promoter.

Other seed-specific genes may also serve as useful sources of promoters. cDNa clones of cruciferin, the other major seed storage protein of B. napus, have been identified (Simon et al., 1985, supra) and could be used to screen a genomic library for promoters.

Without knowing their specific functions, yet other cDNA clones can be classified as to their level of expression in seed tissues, their timing of expression (i.e., when post-anthesis they are expressed) and their approximate representation (copy number) in the B. campestris genome. Clones fitting the criteria necessary for expressing genes related to fatty acid synthesis or other seed functions can be used to screen a genomic library for genomic clones which contain the 5' and 3' regulatory regions necessary for expression. The non-coding regulatory regions can be manipulated to make a tissue-specific expression cassette in the general manner described for the napin genes in previous examples.

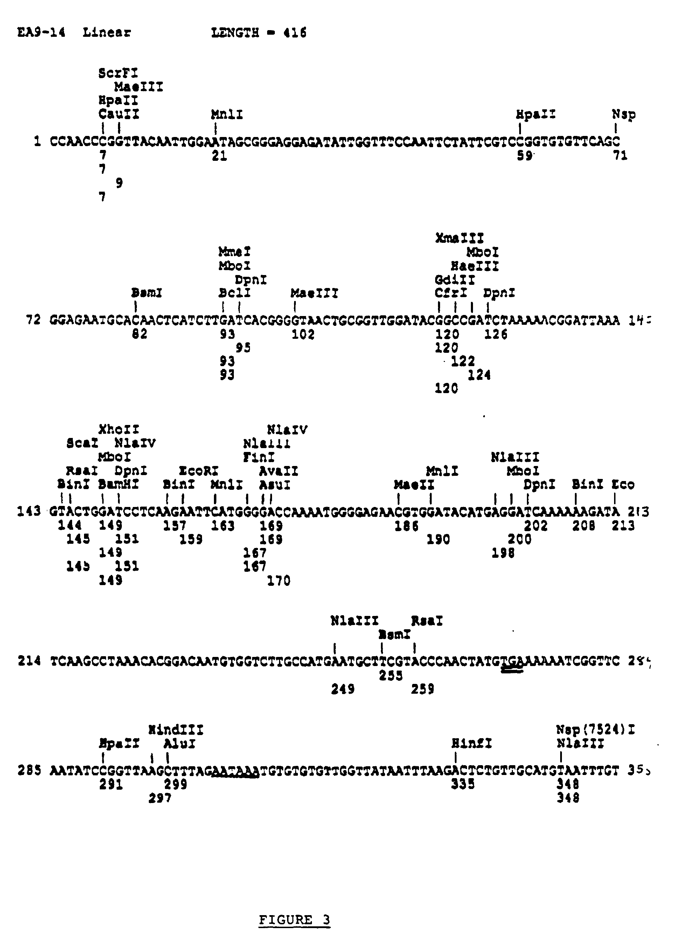

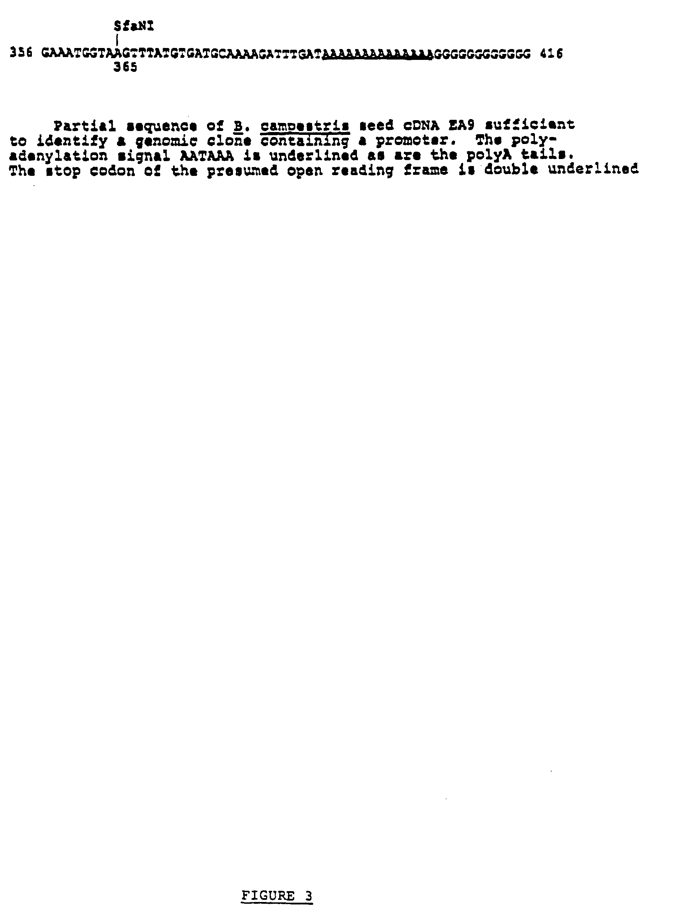

One example of a cDNA clone is EA9. It is highly expressed in seeds and not leaves from B. campestris. It represents a highly abundant mRNA as shown by cross-hybridization of seven other cDNAs from the library by dot blot hybridization. Northern blot analysis of mRNA isolated from day 14 seed, and day 21 and 28 post-anthesis embryos using a 700 bp EcoRI fragment of EA9 as a probe shows that EA9 is highly expressed at day 14 and expressed at a much lower level at day 21 and day 28. The restriction map of EA9 was determined and the clone sequenced. Identification of a polyadenylation signal and of polyA tails at the 3'-end of EA9 confirms the orientation of the cDNA clone and the direction of transcription of the mRNA. The partial sequence provided here for clone EA9 (Figure 3) can be used to synthesize a probe which will identify a unique class of Brassica seed-specific promoters.

It is evident from the above results, that transcription or expression can be obtained specifically in seeds, so as to permit the modulation of phenotype or change in properties of a product of seed, particularly of the embryo. It is found that one can use transcriptional initiation regions associated with the transcription of sequences in seeds in conjunction with sequences other than the normal sequence to produce endogenous or exogenous proteins or modulate the transcription or expression of nucleic acid sequences. In this manner, seeds can be used to produce novel products, to provide for improved protein compositions, to modify the distribution of fatty acid, and the like.

All publicatons and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

## Claims (Claims for the following Contracting State(s): BE, CH, LI, DE, FR, GB, GR, IT, LU, NL, SE)

1. A DNA construct comprising in the 5' to 3' direction of transcription:
a napin transcriptional initiation region, joined to
a DNA sequence of interest other than (i) a DNA sequence encoding napin or (ii) a DNA sequence encoding a mammalian protein or peptide or mammalian viral pathogen peptide or protein; and
a transcription termination region.

2. A DNA construct according to claim 1, wherein said construct further comprises a translational initiation region positioned downstream of said transcription initiation region in the 5' to 3' direction.

3. A DNA construct according to claim 1 or claim 2, wherein said DNA sequence of interest is an open reading frame encoding an amino acid sequence.

4. A DNA construct according to any one of claims 1 to 3 wherein the sequence of transcriptional initiation region is obtainable from Fig. 1 or Fig.2, and comprises a region of about 300 nucleotides 5' of the ATG translation start codon of a napin gene.

5. A DNA construct according to any one of the claims 1 to 4 wherein said transcriptional termination region is from a gene native to said transcriptional initiation region.

6. A DNA construct comprising in the 5' to 3' direction of transcription:
a napin transcriptional initiation region wherein said napin transcriptional initiation region is free from the native DNA sequence under the regulatory control of said initiation region, joined to a cloning site, and a transcriptional termination region;
provided that said construct does not comprise a DNA sequence encoding a mammalian peptide or protein or mammalian viral pathogen peptide or protein operably linked to said napin transcriptional initiation region.

7. A DNA construct according to claim 6 wherein a DNA sequence of interest other than the native sequence is inserted into said cloning site.

8. A method of modifying the genotype of a plant to impart a desired characteristic to seed as distinct from other plant tissues said method comprising:
transforming a host plant cell under genomic integration conditions with a DNA construct comprising in the 5' to 3' direction of transcription:
a seed specific napin transcriptional initiation region, joined to
a DNA sequence of interest other than (a) a DNA sequence encoding a napin or (b) a DNA sequence encoding a mammalian peptide or protein or a mammalian viral pathogen peptide or protein; and
a transcriptional termination region; and
growing said plant to produce seed.

9. A method for specifically modifying the phenotype of seed as distinct from other plant tissue, said method comprising:
(i) transforming a host plant cell under genomic integration conditions with a DNA construct comprising in the 5' to 3' direction of transcription:
a seed specific napin transcriptional initiation region, joined to
a DNA sequence of interest other than (a) a DNA sequence encoding napin or (b) a DNA sequence encoding a mammalian peptide or protein or a mammalian viral pathogen peptide or protein; and
a transcriptional termination region; and
(ii) growing a plant under conditions to produce seed, said plant being comprised of cells capable of developing seed tissue and said cells having integrated in their genome said DNA construct.

10. A DNA construct comprising in the 5' to 3' direction of transcription:
a seed specific transcriptional initiation region which is from other than the bean phaseolin promoter;
a DNA sequence other than the natural coding sequence joined to said initiation region, wherein said sequence encodes an acyl carrier protein; and
a transcriptional termination region.

11. A DNA construct according to claim 10 wherein said transcriptional initiation region is from a napin gene.

12. A method for modifying the genotype of a plant to impart a desired characteristic to seed as distinct from other plant tissues said method comprising:
transforming a host plant cell under genomic integration conditions with a DNA construct comprising in the 5' to 3' direction of transcription:
a seed specific transcriptional initiation region;
a DNA sequence encoding an acyl carrier protein joined to said initiation region;
and
a transcriptional termination region; and
growing said plant to produce seed.

13. A method for specifically modifying the phenotype of seed as distinct from other plant tissue, said method comprising:
(i) transforming a host plant cell under genomic integration conditions with a DNA construct comprising in the 5' to 3' direction of transcription:
a seed specific transcriptional initiation region;
a DNA sequence encoding an acyl carrier protein joined to said initiation region;
and
a transcriptional termination region; and
(ii) growing a plant under conditions to produce seed, said plant being comprised of cells capable of developing seed tissue and said cells having integrated in their genome said DNA construct.

14. A method of modifying the genotype of a plant to impart a desired characteristic to seed as distinct from other plant tissue said method comprising:
transforming a host plant cell under genomic integration conditions with a DNA construct as defined in any one of claims 1 to 7, 10 and 11;
growing said plant to produce seed.

15. A method for specifically modifying the phenotype of seed as distinct from other plant tissue, said method comprising:
(i) transforming a host plant cell under genomic integration conditions with a DNA according to any one of claims 1 to 7, 10 and 11; and
(ii) growing a plant under conditions to produce seed, said plant being comprised of cells capable of developing seed tissue and said cells having integrated in their genome said DNA construct.

16. A method according to any one of claims 8, 9 and 12 to 15 wherein said plant is selected from Brassica, cotton, soybean, safflower and sunflower.

17. A vector comprising a DNA construct according to any one of claims 1 to 7, 10 and 11, a prokaryotic replication system and a marker for selection of transformed prokaryotes comprising said marker.

18. A plant cell comprising a DNA construct according to any one of claims 1 to 7, 10 and 11.

19. A plant cell comprising a DNA construct according to any one of claims 1 to 7, 10 and 11, wherein said cell or seed is from a plant selected from Brassica, cotton, soybean, safflower and sunflower.

## Claims (Claims for the following Contracting State(s): AT, ES)

1. A method comprising the production of a DNA construct comprising in the 5' to 3' direction of transcription:
a napin transcriptional initiation region, joined to
a DNA sequence of interest other than (i) a DNA sequence encoding napin or (ii) a DNA sequence encoding a mammalian protein or peptide or mammalian viral pathogen peptide or protein; and
a transcription termination region.

2. A method according to claim 1, wherein said construct further comprises a translational initiation region positioned downstream of said transcription initiation region in the 5' to 3' direction.

3. A method according to claim 1 or claim 2, wherein said DNA sequence of interest is an open reading frame encoding an amino acid sequence.

4. A method according to any one of claims 1 to 3 wherein the sequence of transcriptional initiation region is obtainable from Fig.1 or Fig.2, and comprises a region of about 300 nucleotides 5' of the ATG translation start codon of a napin gene.

5. A method according to any one of the claims 1 to 4 wherein said transcriptional termination region is from a gene native to said transcriptional initiation region.

6. A method comprising the production of a DNA construct comprising in the 5' to 3' direction of transcription:
a napin transcriptional initiation region wherein said napin transcriptional initiation region is free from the native DNA sequence under the regulatory control of said initiation region, joined to a cloning site, and a transcriptional termination region;
provided that said construct does not comprise a DNA sequence encoding a mammalian peptide or protein or mammalian viral pathogen peptide or protein operably linked to said napin transcriptional initiation region.

7. A method according to claim 6 wherein a DNA sequence of interest other than the native sequence is inserted into said cloning site.

8. A method of modifying the genotype of a plant to impart a desired characteristic to seed as distinct from other plant tissues said method comprising:
transforming a host plant cell under genomic integration conditions with a DNA construct comprising in the 5' to 3' direction of transcription:
a seed specific napin transcriptional initiation region, joined to a DNA sequence of interest other than (a) a DNA sequence encoding a napin or (b) a DNA sequence encoding a mammalian peptide or protein or a mammalian viral pathogen peptide or protein; and
a transcriptional termination region; and
growing said plant to produce seed.

9. A method for specifically modifying the phenotype of seed as distinct from other plant tissue, said method comprising:
(i) transforming a host plant cell under genomic integration conditions with a DNA construct comprising in the 5' to 3' direction of transcription:
a seed specific napin transcriptional initiation region, joined to
a DNA sequence of interest other than (a) a DNA sequence encoding napin or (b) a DNA sequence encoding a mammalian peptide or protein or a mammalian viral pathogen peptide or protein; and
a transcriptional termination region; and
(ii) growing a plant under conditions to produce seed, said plant being comprised of cells capable of developing seed tissue and said cells having integrated in their genome said DNA construct.

10. A method comprising the production of a DNA construct comprising in the 5' to 3' direction of transcription:
a seed specific transcriptional initiation region which is from other than the bean phaseolin promoter;
a DNA sequence other than the natural coding sequence joined to said initiation region, wherein said sequence encodes an acyl carrier protein; and
a transcriptional termination region.

11. A method according to claim 10 wherein said transcriptional initiation region is from a napin gene.

12. A method for modifying the genotype of a plant to impart a desired characteristic to seed as distinct from other plant tissues said method comprising:
transforming a host plant cell under genomic integration conditions with a DNA construct comprising in the 5' to 3' direction of transcription:
a seed specific transcriptional initiation region;
a DNA sequence encoding an acyl carrier protein joined to said initiation region;
and
a transcriptional termination region; and
growing said plant to produce seed.

13. A method for specifically modifying the phenotype of seed as distinct from other plant tissue, said method comprising:
(i) transforming a host plant cell under genomic integration conditions with a DNA construct comprising in the 5' to 3' direction of transcription:
a seed specific transcriptional initiation region;
a DNA sequence encoding an acyl carrier protein joined to said initiation region;
and
a transcriptional termination region; and
(ii) growing a plant under conditions to produce seed, said plant being comprised of cells capable of developing seed tissue and said cells having integrated in their genome said DNA construct.

14. A method of modifying the genotype of a plant to impart a desired characteristic to seed as distinct from other plant tissue said method comprising:
transforming a host plant cell under genomic integration conditions with a DNA construct as defined in any one of claims 1 to 7, 10 and 11;
growing said plant to produce seed.

15. A method for specifically modifying the phenotype of seed as distinct from other plant tissue, said method comprising:
(i) transforming a host plant cell under genomic integration conditions with a DNA according to any one of claims 1 to 7, 10 and 11; and
(ii) growing a plant under conditions to produce seed, said plant being comprised of cells capable of developing seed tissue and said cells having integrated in their genome said DNA construct.

16. A method according to any one of claims 8, 9 and 12 to 15 wherein said plant is selected from Brassica, cotton, soybean, safflower and sunflower.

17. A method comprising the production of a vector comprising a DNA construct according to any one of claims 1 to 7, 10 and 11, a prokaryotic replication system and a marker for selection of transformed prokaryotes comprising said marker.

18. A method comprising the production of a plant cell or seed involving transformation with a DNA construct according to any one of claims 1 to 7, 10 and 11.

19. A method according to claim 18 wherein said seed is from a plant selected from Brassica, cotton, soybean, safflower and sunflower.

## Patentansprüche (Patentansprüche für folgende(n) Vertragsstaat(en): BE, CH, LI, DE, FR, GB, GR, IT, LU, NL, SE)

1. DNA-Konstrukt, umfassend in der 5'-3'-Transkriptionsrichtung:
eine Napin-Transkriptionsinitiationsregion, gebunden an
eine DNA-Sequenz von Interesse, die eine andere ist als (i) eine DNA-Sequenz, die für Napin kodiert, oder (ii) eine DNA-Sequenz, die für ein Säugetierprotein oder -peptid oder virales Erreger-Säugetierpeptid oder -protein kodiert; und
eine Transkriptionsterminationsregion.

2. DNA-Konstrukt nach Anspruch 1, worin das Konstrukt weiters eine Translationsinitiationsregion umfasst, die stromab von der Transkriptionsinitiationsregion in 5'-3'-Richtung positioniert ist.

3. DNA-Konstrukt nach Anspruch 1 oder 2, worin die DNA-Sequenz von Interesse ein offener Leserahmen ist, der für eine Aminosäuresequenz kodiert.

4. DNA-Konstrukt nach einem der Ansprüche 1 bis 3, worin die Sequenz der Transkriptionsinitiationsregion aus den Fig. 1 oder 2 ersichtlich ist, umfassend eine Region von etwa 300 Nukleotiden 5' vom ATG-Translationsstartcodon eines Napingens.

5. DNA-Konstrukt nach einem der Ansprüche 1 bis 4, worin die Transkriptionsterminationsregion aus einem Gen stammt, das für die Transkriptionsinitiationsregion nativ ist.

6. DNA-Konstrukt, umfassend in 5'-3'-Richtung:
eine Napin-Transkriptionsinitiationsregion, worin die Napin-Transkriptionsinitiationsregion frei von der nativen DNA-Sequenz unter der regulatorischen Steuerung der Initiationsregion ist, gebunden an eine Klonierungsstelle, und eine Transkriptionsterminationsregion;
mit der Maßgabe, dass das Konstrukt keine DNA-Sequenz umfasst, die für ein Säugetierpeptid oder -protein oder virales Erreger-Säugetierpeptid oder -protein kodiert, das mit der Napin-Transkriptionsinitiationsregion operabel verbunden ist.

7. DNA-Konstrukt nach Anspruch 6, worin eine andere DNA-Sequenz von Interesse als die Nativsequenz in die Klonierungsstelle insertiert ist.

8. Verfahren zum Modifizieren des Genotyps einer Pflanze, um Samen (zum Unterschied von anderen pflanzlichen Geweben) eine gewünschte Eigenschaft zu verleihen, welches Verfahren die folgenden Schritte umfasst:
Transformieren einer Pflanzenzelle unter genomischen Integrationsbedingungen mit einem DNA-Konstrukt, das in 5'-3'-Transkriptionsrichtung Folgendes umfasst:
eine samenspezifische Napin-Transkriptionsinitiationsregion, gebunden an
eine DNA-Sequenz von Interesse, die eine andere ist als (a) eine DNA-Sequenz, die für ein Napin kodiert, oder (b) eine DNA-Sequenz, die für ein Säugetierpeptid oder -protein oder ein virales Erreger-Säugetierpeptid oder -protein kodiert; und
ein virales Erregerpeptid oder -protein; und
eine Transkriptionsterminationsregion; und
Züchten der Pflanze, um Samen zu erzeugen.

9. Verfahren zum spezifischen Modifizieren des Phenotyps von Samen (zum Unterschied von anderem Pflanzengewebe), welches Verfahren die folgenden Schritte umfasst:
(i) Transformieren einer Wirtspflanzenzelle unter genomischen Integrationsbedingungen mit einem DNA-Konstrukt, das in 5'-3'-Transkriptionsrichtung Folgendes umfasst:
eine samenspezifische Napin-Transkriptionsinitiationsregion, gebunden an
eine DNA-Sequenz von Interesse, die eine andere ist als (a) eine für Napin kodierende DNA-Sequenz oder (b) eine DNA-Sequenz, die für ein Säugetierpeptid oder -protein oder ein virales Erreger-Säugetierpeptid oder-protein kodiert; und
eine Transkriptionsterminationsregion; und
(ii) Züchten einer Pflanze unter Bedingungen zur Erzeugung von Samen, wobei die Pflanze aus Zellen besteht, die Samengewebe entwickeln können und in ihr Genom das DNA-Konstrukt integriert haben.

10. DNA-Konstrukt, umfassend in 5'-3'-Transkriptionsrichtung:
eine samenspezifische Transkriptionsinitiationsregion, die aus einem anderen als dem Bohnen-Phaseolin-Promotor stammt;
eine DNA-Sequenz, die eine andere als die natürliche Kodiersequenz ist, gebunden an die Initiationsregion, worin die Sequenz für ein Acyl-Trägerprotein kodiert; und
eine Transkriptionsterminationsregion.

11. DNA-Konstrukt nach Anspruch 10, worin die Transkriptionsinitiationsregion aus einem Napingen stammt.

12. Verfahren zum Modifizieren des Genotyps einer Pflanze, um Samen (zum Unterschied von anderen pflanzlichen Geweben) eine gewünschte Eigenschaft zu verleihen, welches Verfahren die folgenden Schritte umfasst:
Transformieren einer Wirtspflanzenzelle unter genomischen Integrationsbedingungen mit einem DNA-Konstrukt, umfassend in 5'-3'-Transkriptionsrichtung:
eine samenspezifische Transkriptionsinitiationsregion;
eine DNA-Sequenz, die für ein Acyl-Trägerprotein kodiert, gebunden an die Initiationsregion; und
eine Transkriptionsterminationsregion; und
Züchten der Pflanze, um Samen zu erzeugen.

13. Verfahren zum spezifischen Modifizieren des Phenotyps von Samen (zum Unterschied von anderem Pflanzengewebe), welches Verfahren die folgenden Schritte umfasst:
(i) Transformieren einer Wirtspflanzenzelle unter genomischen Integrationsbedingungen mit einem DNA-Konstrukt, das in 5'-3'-Transkriptionsrichtung Folgendes umfasst:
eine samenspezifische Transkriptionsinitiationsregion;
eine DNA-Sequenz, die für ein Acyl-Trägerprotein kodiert, gebunden an die Initiationsregion; und
eine Transkriptionsterminationsregion; und
(ii) Züchten einer Pflanze unter Bedingungen, um Samen zu erzeugen, wobei die Pflanze aus Zellen besteht, die Samengewebe entwickeln können und in ihr Genom ein DNA-Konstrukt integriert haben.

14. Verfahren zum Modifizieren des Genotyps einer Pflanze, um Samen (zum Unterschied von anderem Pflanzengewebe) eine gewünschte Eigenschaft zu verleihen, wobei das Verfahren folgende Schritte umfasst:
Transformieren einer Wirtspflanzenzelle unter genomischen Integrationsbedingungen mit einemDNA-Konstrukt nach einem der Ansprüche 1 bis 7, 10 und 11;
Züchten der Pflanze, um Samen zu erzeugen.

15. Verfahren zum spezifischen Modifizieren des Phenotyps von Samen (zum Unterschied von anderem Pflanzengewebe), wobei das Verfahren folgende Schritte umfasst:
(i) Transformieren einer Pflanzenzelle unter genomischen Integrationsbedingungen mit einer DNA nach einem der Ansprüche 1 bis 7, 10 und 11; und
(ii) Züchten einer Pflanze unter Bedingungen, um Samen zu erzeugen, wobei die Pflanze aus Zellen besteht, die Samengewebe entwickeln können und in ihr Genom das DNA-Konstrukt integriert haben.

16. Verfahren nach einem der Ansprüche 8, 9 und 12 bis 15, worin die Pflanze aus Brassica, Baumwolle, Sojabohnen, Safflor und Sonnenblume ausgewählt ist.

17. Vektor, umfassend ein DNA-Konstrukt nach einem der Ansprüche 1 bis 7, 10 und 11, ein prokaryotisches Replikationssystem und einen Marker für die Selektion transformierter Prokaryoten, die den Marker umfassen.

18. Pflanzenzelle, umfassend ein DNA-Konstrukt nach einem der Ansprüche 1 bis 7, 10 und 11.

19. Pflanzenzelle, umfassend ein DNA-Konstrukt nach einem der Ansprüche 1 bis 7, 10 und 11, worin die Zelle oder der Samen aus einer Pflanze stammt, die aus Brassica, Baumwolle, Sojabohnen, Safflor und Sonnenblume ausgewählt ist.

## Patentansprüche (Patentansprüche für folgende(n) Vertragsstaat(en): AT, ES)

1. Verfahren zur Produktion eines DNA-Konstrukts, umfassend in der 5'-3'-Transkriptionsrichtung:
eine Napin-Transkriptionsinitiationsregion, gebunden an
eine DNA-Sequenz von Interesse, die eine andere ist als (i) eine DNA-Sequenz, die für Napin kodiert, oder (ii) eine DNA-Sequenz, die für ein Säugetierprotein oder -peptid oder virales Erreger-Säugetierpeptid oder -protein kodiert; und
eine Transkriptionsterminationsregion.

2. Verfahren nach Anspruch 1, worin das Konstrukt weiters eine Translationsinitiationsregion umfasst, die stromab von der Transkriptionsinitiationsregion in 5'-3'-Richtung positioniert ist.

3. Verfahren nach Anspruch 1 oder 2, worin die DNA-Sequenz von Interesse ein offener Leserahmen ist, der für eine Aminosäuresequenz kodiert.

4. Verfahren nach einem der Ansprüche 1 bis 3, worin die Sequenz der Transkriptionsinitiationsregion aus den Fig. 1 oder 2 ersichtlich ist, umfassend eine Region von etwa 300 Nukleotiden 5' vom ATG-Translationsstartcodon eines Napingens.

5. Verfahren nach einem der Ansprüche 1 bis 4, worin die Transkriptionsterminationsregion aus einem Gen stammt, das für die Transkriptionsinitiationsregion nativ ist.

6. Verfahren zur Produktion eines DNA-Konstrukts, umfassend in 5'-3'-Richtung:
eine Napin-Transkriptionsinitiationsregion, worin die Napin-Transkriptionsinitiationsregion frei von der nativen DNA-Sequenz unter der regulatorischen Steuerung der Initiationsregion ist, gebunden an eine Klonierungsstelle, und eine Transkriptionsterminationsregion;
mit der Maßgabe, dass das Konstrukt keine DNA-Sequenz umfasst, die für ein Säugetierpeptid oder -protein oder virales Erreger-Säugetierpeptid oder -protein kodiert, das mit der Napin-Transkriptionsinitiationsregion operabel verbunden ist.

7. Verfahren nach Anspruch 6, worin eine andere DNA-Sequenz von Interesse als die Nativsequenz in die Klonierungsstelle insertiert ist.

8. Verfahren zum Modifizieren des Genotyps einer Pflanze, um Samen (zum Unterschied von anderen pflanzlichen Geweben) eine gewünschte Eigenschaft zu verleihen, welches Verfahren die folgenden Schritte umfasst:
Transformieren einer Pflanzenzelle unter genomischen Integrationsbedingungen mit einem DNA-Konstrukt, das in 5'-3'-Transkriptionsrichtung Folgendes umfasst:
eine samenspezifische Napin-Transkriptionsinitiationsregion, gebunden an
eine DNA-Sequenz von Interesse, die eine andere ist als (a) eine DNA-Sequenz, die für ein Napin kodiert, oder (b) eine DNA-Sequenz, die für ein Säugetierpeptid oder -protein oder ein virales Erreger-Säugetierpeptid oder -protein kodiert; und
ein virales Erregerpeptid oder -protein; und
eine Transkriptionsterminationsregion; und
Züchten der Pflanze, um Samen zu erzeugen.

9. Verfahren zum spezifischen Modifizieren des Phenotyps von Samen (zum Unterschied von anderem Pflanzengewebe), welches Verfahren die folgenden Schritte umfasst:
(i) Transformieren einer Wirtspflanzenzelle unter genomischen Integrationsbedingungen mit einem DNA-Konstrukt, das in 5'-3'-Transkriptionsrichtung Folgendes umfasst:
eine samenspezifische Napin-Transkriptionsinitiationsregion, gebunden an
eine DNA-Sequenz von Interesse, die eine andere ist als (a) eine für Napin kodierende DNA-Sequenz oder (b) eine DNA-Sequenz, die für ein Säugetierpeptid oder -protein oder ein virales Erreger-Säugetierpeptid oder -protein kodiert; und
eine Transkriptionsterminationsregion; und
(ii) Züchten einer Pflanze unter Bedingungen zur Erzeugung von Samen, wobei die Pflanze aus Zellen besteht, die Samengewebe entwickeln können und in ihr Genom das DNA-Konstrukt integriert haben.

10. Verfahren zur Produktion eines DNA-Konstrukts, umfassend in 5'-3'-Transkriptionsrichtung:
eine samenspezifische Transkriptionsinitiationsregion, die aus einem anderen als dem Bohnen-Phaseolin-Promotor stammt;
eine DNA-Sequenz, die eine andere als die natürliche Kodiersequenz ist, gebunden an die Initiationsregion, worin die Sequenz für ein Acyl-Trägerprotein kodiert; und
eine Transkriptionsterminationsregion.

11. Verfahren nach Anspruch 10, worin die Transkriptionsinitiationsregion aus einem Napingen stammt.

12. Verfahren zum Modifizieren des Genotyps einer Pflanze, um Samen (zum Unterschied von anderen pflanzlichen Geweben) eine gewünschte Eigenschaft zu verleihen, welches Verfahren die folgenden Schritte umfasst:
Transformieren einer Wirtspflanzenzelle unter genomischen Integrationsbedingungen mit einem DNA-Konstrukt, umfassend in 5'-3'-Transkriptionsrichtung:
eine samenspezifische Transkriptionsinitiationsregion;
eine DNA-Sequenz, die für ein Acyl-Trägerprotein kodiert, gebunden an die Initiationsregion; und
eine Transkriptionsterminationsregion; und
Züchten der Pflanze, um Samen zu erzeugen.

13. Verfahren zum spezifischen Modifizieren des Phenotyps von Samen (zum Unterschied von anderem Pflanzengewebe), welches Verfahren die folgenden Schritte umfasst:
(i) Transformieren einer Wirtspflanzenzelle unter genomischen integrationsbedingungen mit einem DNA-Konstrukt, das in 5'-3'-Transkriptionsrichtung Folgendes umfasst:
eine samenspezifische Transkriptionsinitiationsregion;
eine DNA-Sequenz, die für ein Acyl-Trägerprotein kodiert, gebunden an die Initiationsregion; und
eine Transkriptionsterminationsregion; und
(ii) Züchten einer Pflanze unter Bedingungen, um Samen zu erzeugen, wobei die Pflanze aus Zellen besteht, die Samengewebe entwickeln können und in ihr Genom ein DNA-Konstrukt integriert haben.

14. Verfahren zum Modifizieren des Genotyps einer Pflanze, um Samen (zum Unterschied von anderem Pflanzengewebe) eine gewünschte Eigenschaft zu verleihen, wobei das Verfahren folgende Schritte umfasst:
Transformieren einer Wirtspflanzenzelle unter genomischen Integrationsbedingungen mit einemDNA-Konstrukt nach einem der Ansprüche 1 bis 7, 10 und 11;
Züchten der Pflanze, um Samen zu erzeugen.

15. Verfahren zum spezifischen Modifizieren des Phenotyps von Samen (zum Unterschied von anderem Pflanzengewebe), wobei das Verfahren folgende Schritte umfasst:
(i) Transformieren einer Pflanzenzelle unter genomischen Integrationsbedingungen mit einer DNA nach einem der Ansprüche 1 bis 7, 10 und 11; und
(ii) Züchten einer Pflanze unter Bedingungen, um Samen zu erzeugen, wobei die Pflanze aus Zellen besteht, die Samengewebe entwickeln können und in ihr Genom das DNA-Konstrukt integriert haben.

16. Verfahren nach einem der Ansprüche 8, 9 und 12 bis 15, worin die Pflanze aus Brassica, Baumwolle, Sojabohnen, Safflor und Sonnenblume ausgewählt ist.

17. Vektor, umfassend ein DNA-Konstrukt nach einem der Ansprüche 1 bis 7, 10 und 11, ein prokaryotisches Replikationssystem und einen Marker für die Selektion transformierter Prokaryoten, die den Marker umfassen.

18. Verfahren zur Produktion einer Pflanzenzelle oder von Pflanzensamen, umfassend die Transformation mit einem DNA-Konstrukt nach einem der Ansprüche 1 bis 7, 10 und 11.

19. Verfahren nach Anspruch 18, worin der Samen von einer Pflanze stammt, die aus Brassica, Baumwolle, Sojabohnen, Safflor und Sonnenblume ausgewählt ist.

## Revendications (Revendications pour l'(les) Etat(s) contractant(s) suivant(s): BE, CH, LI, DE, FR, GB, GR, IT, LU, NL, SE)

1. Construction d'ADN comprenant dans la direction 5' à 3' de transcription :
une région d'initiation de transcription de napine, jointe à
une séquence d'ADN d'intérêt autre que (i) une séquence d'ADN codant pour la napine, ou (ii) une séquence d'ADN codant pour une protéine mammalienne ou peptide, ou peptide pathogène viral mammalien ou protéine ; et
une région de terminaison de transcription.

2. Construction d'ADN selon la revendication 1, où ladite construction comprend, de plus, une région d'initiation de traduction placée en aval de ladite région d'initiation de transcription dans la direction 5' à 3'.

3. Construction d'ADN selon la revendication 1 ou la revendication 2, où ladite séquence d'ADN d'intérêt est un cadre de lecture ouvert codant pour une séquence d'acides aminés.

4. Construction d'ADN selon l'une quelconque des revendications 1 à 3, où la séquence de la région d'initiation de transcription peut être obtenue de la figure 1 ou de la figure 2, et comprend une région d'environ 300 nucléotides à 5' du codon de début de traduction ATG d'un gène de napine.

5. Construction d'ADN selon l'une quelconque des revendications 1 à 4, où ladite région de terminaison de transcription est d'un gène natif à ladite région d'initiation de transcription.

6. Construction d'ADN comprenant dans la direction 5' à 3' de transcription :
une région d'initiation de transcription de napine où ladite région d'initiation de transcription de napine est exempte de la séquence d'ADN native sous le contrôle de régulation de ladite région d'initiation, jointe à un site de clonage et une région de terminaison de transcription ;
à condition que ladite construction ne comprenne pas une séquence d'ADN codant pour un peptide mammalien ou protéine ou un peptide pathogène viral mammalien ou protéine, opérativement enchaîné à ladite région d'initiation de transcription de la napine.

7. Construction d'ADN selon la revendication 6, où une séquence d'ADN d'intérêt autre que la séquence native est insérée dans ledit site de clonage.

8. Méthode de modification du génotype d'une plante pour impartir une caractéristique souhaitée à la graine, distincte d'autres tissus de plante, ladite méthode comprenant :
la transformation d'une cellule d'une plante hôte en conditions d'intégration génomique avec une construction d'ADN comprenant dans la direction 5' à 3' de transcription :
une région d'initiation de transcription de napine spécifique de la graine, jointe à
une séquence d'ADN d'intérêt autre que (a) une séquence d'ADN codant pour une napine ou (b) une séquence d'ADN codant pour un peptide mammalien ou protéine, ou un peptide pathogène viral mammalien ou protéine ; et
une région de terminaison de transcription ; et
la croissance de ladite plante produire une graine.

9. Méthode pour modifier spécifiquement le phénotype d'une graine distincte d'un autre tissu de plante, ladite méthode comprenant :
(i) la transformation d'une cellule de plante hôte en conditions d'intégration génomique avec une construction d'ADN comprenant dans la direction 5' à 3' de transcription :
une région d'initiation de transcription de la napine spécifique de la graine, jointe à
une séquence d'ADN d'intérêt autre que (a) une séquence d'ADN codant pour la napine ou (b) une séquence d'ADN codant pour un peptide mammalien ou protéine, ou un peptide pathogène viral mammalien ou protéine ; et
une région de terminaison de transcription ; et
(ii) la croissance de ladite plante dans des conditions pour produire une graine, ladite plante se composant de cellules capables de développer du tissu de graine, les lesdites cellules ayant, intégrée dans leur génome, ladite construction d'ADN.

10. Construction d'ADN comprenant dans la direction 5' à 3' de transcription :
une région d'initiation de transcription spécifique de la graine qui est d'autres que le promoteur de phaséoline du haricot ;
une séquence d'ADN autre que la séquence codante naturelle jointe à ladite région d'initiation, où ladite séquence code pour une protéine porteuse d'acyle ; et
une région de terminaison de transcription.

11. Construction d'ADN selon la revendication 10, où ladite région d'initiation de transcription est d'un gène de napine.

12. Méthode pour modifier le génotype d'une plante pour impartir une caractéristique souhaitée à une graine distincte d'autres tissus de plante, ladite méthode comprenant :
la transformation d'une cellule de plante hôte en conditions d'intégration génomique avec une construction d'ADN comprenant dans la direction 5' à 3' de transcription :
une région d'initiation de transcription de napine spécifique de la graine ;
une séquence d'ADN codant pour une protéine porteuse d'acyle jointe à ladite région d'initiation ; et
une région de terminaison de transcription ; et la croissance de ladite plante produire une graine.

13. Méthode pour modifier spécifiquement le phénotype d'une graine distincte d'autres tissus de plante, ladite méthode comprenant :
(i) la transformation d'une cellule d'une plante hôte en conditions d'intégration génomique avec une construction d'ADN comprenant dans la direction 5' à 3' de transcription :
une région d'initiation de transcription spécifique de la graine ;
une séquence d'ADN codant pour une protéine porteuse d'acyle jointe à ladite région d'initiation ; et
une région de terminaison de transcription ; et
(ii) la croissance d'une plante dans des conditions pour produire une graine, ladite plante se composant de cellules capables de développer le tissu de la graine, et lesdites cellules ayant, intégrée dans leur génome, ladite construction d'ADN.

14. Méthode de modification du génotype d'une plante pour impartir une caractéristique souhaitée à la graine, distincte d'un autre tissu de la plante, ladite méthode comprenant :
la transformation d'une cellule de plante hôte en conditions d'intégration génomique avec une construction d'ADN telle que définie dans l'une quelconque des revendications 1 à 7, 10 et 11 ;
la croissance de ladite plante pour produire une graine.

15. Méthode pour modifier spécifiquement le phénotype d'une graine distincte d'un autre tissu de la plante, ladite méthode comprenant :
(i) la transformation d'une cellule de plante hôte en conditions d'intégration génomique avec un ADN selon l'une quelconque des revendications 1 à 7, 10 et 11 ; et
(ii) la croissance d'une plante dans des conditions pour produire une graine, ladite plante se composant de cellules capables de développer le tissu de la graine, et lesdites cellules ayant, intégrée dans leur génome, ladite construction d'ADN.

16. Méthode selon l'une quelconque des revendications 8, 9 et 12 à 15, où ladite plante est sélectionnée parmi Brassica, coton, soja, carthame et tournesol.

17. Vecteur comprenant une construction d'ADN selon l'une quelconque des revendications 1 à 7, 10 et 11, un système de réplication procariote et un marqueur pour la sélection des procariotes transformés comprenant ledit marqueur.

18. Cellule de plante comprenant une construction d'ADN selon l'une quelconque des revendications 1 à 7, 10 et 11.

19. Cellule de plante comprenant une construction d'ADN selon l'une quelconque des revendications 1 à 7, 10 et 11, où ladite cellule ou graine est d'une plante sélectionnée parmi Brassica, coton, soja, carthame et tournesol.

## Revendications (Revendications pour l'(les) Etat(s) contractant(s) suivant(s): AT, ES)

1. Méthode comprenant la production d'une construction d'ADN comprenant dans la direction 5' à 3' de transcription :
une région d'initiation de transcription de napine, jointe à
une séquence d'ADN d'intérêt autre que (i) une séquence d'ADN codant pour la napine, ou (ii) une séquence d'ADN codant pour une protéine mammalienne ou peptide, ou peptide pathogène viral mammalien ou protéine ; et
une région de terminaison de transcription.

2. Méthode selon la revendication 1, où ladite construction comprend, de plus, une région d'initiation de traduction placée en aval de ladite région d'initiation de transcription dans la direction 5' à 3'.

3. Méthode selon la revendication 1 ou 2, où ladite séquence d'ADN d'intérêt est un cadre de lecture ouvert codant pour une séquence d'acides aminés.

4. Méthode selon l'une quelconque des revendications 1 à 3, où la séquence de la région d'initiation de transcription peut être obtenue de la figure 1 ou de la figure 2, et comprend une région d'environ 300 nucléotides à 5' du codon de début de traduction ATG d'un gène de napine.

5. Méthode selon l'une quelconque des revendications 1 à 4, où ladite région de terminaison de transcription est d'un gène natif à ladite région d'initiation de transcription.

6. Méthode comprenant la production d'une construction d'ADN comprenant dans la direction 5' à 3' de transcription :
une région d'initiation de transcription de napine où ladite région d'initiation de transcription de napine est exempte de la séquence d'ADN native sous le contrôle de régulation de ladite région d'initiation, jointe à un site de clonage et une région de terminaison de transcription ;
à condition que ladite construction ne comprenne pas une séquence d'ADN codant pour un peptide mammalien ou protéine ou un peptide pathogène viral mammalien ou protéine, opérativement enchaîné à ladite région d'initiation de transcription de la napine.

7. Méthode selon la revendication 6, où une séquence d'ADN d'intérêt autre que la séquence native est insérée dans ledit site de clonage.

8. Méthode de modification du génotype d'une plante pour impartir une caractéristique souhaitée à la graine, distincte d'autres tissus de plante, ladite méthode comprenant :
la transformation d'une cellule d'une plante hôte en conditions d'intégration génomique avec une construction d'ADN comprenant dans la direction 5' à 3' de transcription :
une région d'initiation de transcription de napine spécifique de la graine jointe à
une séquence d'ADN d'intérêt autre que (a) une séquence d'ADN codant pour une napine ou (b) une séquence d'ADN codant pour un peptide mammalien ou protéine, ou un peptide pathogène viral, mammalien. ou protéine ; et
une région de terminaison de transcription ; et
la croissance de ladite plante produire une graine.

9. Méthode pour modifier spécifiquement le phénotype d'une graine distincte d'un autre tissu de plante, ladite méthode comprenant :
(i) la transformation d'une cellule de plante hôte en conditions d'intégration génomique avec une construction d'ADN comprenant dans la direction 5' à 3' de transcription :
une région d'initiation de transcription de la napine spécifique de la graine jointe à
une séquence d'ADN d'intérêt autre que (a) une séquence d'ADN codant pour la napine ou (b) une séquence d'ADN codant pour un peptide mammalien ou protéine, ou un peptide pathogène viral, mammalien ou protéine ; et
une région de terminaison de transcription ; et
(ii) la croissance de ladite plante dans des conditions pour produire une graine, ladite plante se composant de cellules capables de développer du tissu de graine, les lesdites cellules ayant, intégrée dans leur génome, ladite construction d'ADN.

10. Méthode comprenant la production d'une construction d'ADN comprenant dans la direction 5' à 3' de transcription :
une région d'initiation de transcription spécifique de la graine qui est d'autres que le promoteur de phaséoline du haricot ;
une séquence d'ADN autre que la séquence codante naturelle jointe à ladite région d'initiation, où ladite séquence code pour une protéine porteuse d'acyle ; et
une région de terminaison de transcription.

11. Méthode selon la revendication 10, où ladite région d'initiation de transcription est d'un gène de napine.

12. Méthode pour modifier le génotype d'une plante pour impartir une caractéristique souhaitée à une graine distincte d'autres tissus de plante, ladite méthode comprenant :
la transformation d'une cellule de plante hôte en conditions d'intégration génomique avec une construction d'ADN comprenant dans la direction 5' à 3' de transcription :
une région d'initiation de transcription de napine spécifique de la graine ;
une séquence d'ADN codant pour une protéine porteuse d'acyle jointe à ladite région d'initiation ; et
une région de terminaison de transcription ; et
la croissance de ladite plante produire une graine.

13. Méthode pour modifier spécifiquement le phénotype d'une graine distincte d'autres tissus de plante, ladite méthode comprenant :
(i) la transformation d'une cellule d'une plante hôte en conditions d'intégration génomique avec une construction d'ADN comprenant dans la direction 5' à 3' de transcription :
une région d'initiation de transcription spécifique de la graine ;
une séquence d'ADN codant pour une protéine porteuse d'acyle jointe à ladite région d'initiation ; et
une région de terminaison de transcription ; et
(ii) la croissance d'une plante dans des conditions pour produire une graine, ladite plante se composant de cellules capables de développer le tissu de la graine, et lesdites cellules ayant, intégrée dans leur génome, ladite construction d'ADN.

14. Méthode de modification du génotype d'une plante pour impartir une caractéristique souhaitée à la graine, distincte d'un autre tissu de la plante, ladite méthode comprenant :
la transformation d'une cellule de plante hôte en conditions d'intégration génomique avec une construction d'ADN telle que définie dans l'une quelconque des revendications 1 à 7, 10 et 11 ;
la croissance de ladite plante pour produire une graine.

15. Méthode pour modifier spécifiquement le phénotype d'une graine distincte d'un autre tissu de la plante, ladite méthode comprenant :
(i) la transformation d'une cellule de plante hôte en conditions d'intégration génomique avec un ADN selon l'une quelconque des revendications 1 à 7, 10 et 11 ; et
(ii) la croissance d'une plante dans des conditions pour produire une graine, ladite plante se composant de cellules capables de développer le tissu de la graine, et lesdites cellules ayant, intégrée dans leur génome, ladite construction d'ADN.

16. Méthode selon l'une quelconque des revendications 8, 9 et 12 à 15, où ladite plante est sélectionnée parmi Brassica, coton, soja, carthame et tournesol.

17. Méthode comprenant la production d'un vecteur comprenant une construction d'ADN selon l'une quelconque des revendications 1 à 7, 10 et 11, un système de réplication procariote et un marqueur pour la sélection des procariotes transformés comprenant ledit marqueur.

18. Méthode comprenant la production d'une cellule de plante ou graine impliquant la transformation avec une construction d'ADN selon l'une quelconque des revendications 1 à 7, 10 et 11.

19. Méthode selon la revendication 18, où ladite graine provient d'une plante sélectionnée parmi Brassica, coton, soja, carthame et tournesol.
